Europäisches Patentamt

European Patent Office

Office européen des brevets

(19)

(11) **EP 1 144 995 B1**

(12) **EUROPEAN PATENT SPECIFICATION**

(45) Date of publication and mention
of the grant of the patent:
**07.04.2004 Bulletin 2004/15**

(21) Application number: **00904362.1**

(22) Date of filing: **14.01.2000**

(51) Int Cl.⁷: **G01N 33/00**

(86) International application number:
**PCT/US2000/000966**

(87) International publication number:
**WO 2000/042427 (20.07.2000 Gazette 2000/29)**

(54) **DETECTION OF BASE CONTAMINANTS IN GAS SAMPLES**

NACHWEIS VON BASISCHEN VERUNREINIGUNGEN IN GASPROBEN

DETECTION DE CONTAMINANTS DE BASE DANS DES ECHANTILLONS GAZEUX

(84) Designated Contracting States:
**AT BE CH CY DE DK ES FI FR GB GR IE IT LI LU MC NL PT SE**

(30) Priority: **14.01.1999 US 232199**

(43) Date of publication of application:
**17.10.2001 Bulletin 2001/42**

(73) Proprietor: **Extraction Systems, Inc.**
**Franklin, MA 02038-3137 (US)**

(72) Inventors:
• **KISHKOVICH, Oleg, P.**
**Greenville, RI 02828 (US)**
• **GOODWIN, William, M.**
**Medway, MA 02053 (US)**
• **PHELPS, Mark**
**Attleboro, MA 02703 (US)**
• **KINKEAD, Devon**
**Holliston, MA 01746 (US)**

(74) Representative: **Greenwood, John David et al**
**Graham Watt & Co.**
**St. Botolph's House**
**7-9 St. Botolph's Road**
**Sevenoaks Kent TN13 3AJ (GB)**

(56) References cited:
| WO-A-98/38508 | US-A- 3 620 931 |
| US-A- 4 333 735 | US-A- 4 335 073 |
| US-A- 4 526 755 | US-A- 5 283 199 |
| US-A- 5 457 316 | US-A- 5 563 330 |
| US-A- 5 739 038 | US-A- 5 835 974 |
| US-A- 5 841 022 | |

## Description

BACKGROUND OF THE INVENTION

The invention relates to the detection of base contaminants in a gas sample, especially amine contaminants, and to systems employing such detection, including semiconductor fabrication systems and systems for filtering gases for semiconductor fabrication and other processes that require uncontaminated atmospheres of high quality.

A particular purpose of the invention is to reliably measure low concentrations of airborne base contaminants in a semiconductor manufacturing environment that may adversely affect base-sensitive photolithographic processes being employed.

In semiconductor manufacturing it has been found desirable to detect airborne basic compounds such as normal methyl pyrrolidinone (NMP) and ammonia. Such contaminants may interfere, for instance, with a photolithography process used in semiconductor fabrication. The base contaminant may react with protons produced as a result of exposure of a photoresist layer to light. This can interfere with proper exposure and development and can harm the yield of the process and the rate of produation of the semiconductor wafers.

For this reason, semiconductor manufacturers have sought to measure and control the concentration of airborne molecular contamination during the critical steps of the photolithography process that are sensitive to it. A detecting instrument specific to the detection of NMP and a detecting instrument specific to the detection of ammonia have been employed in semiconductor manufacturing facilities to monitor the atmospheric quality in the vicinity of production tools.

To understand the novel aspects of the invention it is useful to mention some detection techniques that have been used in other contexts.

For study of combustion processes or atmospheric pollution, a process has been developed for measuring the total fixed gaseous nitrogen species, including $NH_3$, $NO_3$, $NO_2$, HCN and organic amines in gaseous mixtures. The process involves catalytic conversion at elevated temperature of all fixed nitrogen species to NO, followed by chemiluminescent measurement of the resulting NO concentration.

For detection of ammonia, NO and $NO_x$, machines have been made that employ an ammonia scrubber or absorber coupled with a thermal/catalytic converter with or without a molybdenum catalyst. For instance, in one instrument for stack gas analysis, a diluted sample is directed by a valve to alternately flow through or past an absorber that specifically removes ammonia. The alternating samples proceed along a common line through a thermal converter to a chemiluminescent detector that operates in the 650-750 millibar range. By subtracting signals, the ammonia concentration can be calculated.

Another aspect of the invention relates to the use of air filters for the ambient air in semiconductor manufacturing. To avoid harm to the process from NMP or ammonia, semiconductor manufacturers have used chemical filters to remove the contaminants. These filtering systems employ filter stages within an enclosure, the filter media of each stage being penetrable by air with acceptable pressure drop. As air flows through the filtering system, unwanted contaminants are retained on the chemically-active surface of the various stages of the filter system. A problem associated with such filtering systems has been to accurately predict the remaining life of the filter so that the filter media can be changed at appropriate times with minimal disruption to the use of the expensive production facility. In the case of semiconductor fabrication facilities, typically, filter life has been estimated by measuring the concentration of ammonia in the air flow associated with the filter system.

WO-A-98/38508 discloses a detection system constructed to examine multiple amines in gas to produce a reading that is stoichiometrically related to the proton bonding characteristic of the multiple amines present in the sampled gas. In one , aspect, the detection system includes at least two channels through which gas samples to be examined pass, at least one convertor for converting the multiple amines into NO, and at least one chemiluminescent NO detector for producing signals representative of the NO concentration in air passing therethrough. In another aspect, the detection system includes at least two channels through which gas samples to be examined pass, an amine remover, which has a high removal efficency for both strong and weak amines, coupled to one of the channels.

US-A-5,739,038 discloses a gas analyzer system for providing a spectroscopic analysis of the sample gas. This analysis is accomplished by first introducing the sample gas into the inlet of the system and transporting it to a spectral analyzer. The sample gas is then spectrally analyzed and the analyzer outputs a signal indicative of a radiation intensity spectrum associated with the analyzed sample gas. A processing unit uses the analyzer signal to detect the presence of one or more prescribed gases and to determine the concentration of each of the prescribed gases in the sample gas. Next, the reacting agent is supplied to the sample gas to convert one or more gases whose presence in the sample gas cannot be detected via spectral analysis due to the masking effects other gases present in the sample gas. The masked gases are converted to secondary gases at least one of which is readily detectable via spectral analysis. This converting process occurs in the time it takes for the sample gas to transit uninterrupted through the system to the analyzer. The modified sample gas is spectrally analyzed, and the processing unit detects the presence of the masked gases and determines the concentration of each. The system may be employed to monitor certain environmental gases contained within industrial emission. In such a case, the prescribed gases are nitrogen dioxide and sulfur dioxide, and the masked gases are nitric oxide, ammonia, and hydrogen sulfide.

US-A-5,457,864 discloses an ion mobility spectrometer sensor apparatus which is in a hermetically sealed housing, utilizing a drift gas for the determination of trace contaminants in a carrier gas, including a container for a sample gas containing an analyte the concentration of which is to be determined, means for purifying the sample gas to produce the carrier gas from it, the means for purifying being hermetically connected from the container through a metallic pipe, a source for the purified drift gas which may be the same or different than the carrier gas, an ion mobility spectrometer sensor having a carrier gas entrance and a drift gas entrance and a gas exit, the ion mobility spectrometer sensor being hermetically connected by a metallic pipe from the purifying means and from the source of the drift gas, a back diffusion trap is hermetically connected from the gas exit, and a signal readout is electrically and hermetically connected from the ion mobility spectrometer sensor for electrically sensing and displaying signals obtained in the sensor.

The measurement of ammonia, exclusive of other basic contaminants, is unsatisfactory in photolithographic processes that are affected by low concentrations of any basic contaminant gas. One process that is sensitive to low levels of any basic contaminant gas is chemically-amplified deep-ultraviolet (DUV) photoresist processing. Typically, most or all of such basic contaminants that can affect the process include nitrogen. Measurement of total fixed-nitrogen species is not applicable, however, because many of the fixed-nitrogen species ($e.g.$, HCN, NO, $NO_2$) are not basic in nature and do not affect the process.

None of the techniques mentioned above have suggested a concept behind the present invention involving measuring, in a non-specific reading, a low-level concentration of multiple basic nitrogen compounds in a gas sample exposed to photolithographic processes and the like.

The present invention is a detecting system for detecting basic nitrogen compounds, a span-calibrated method for monitoring basic-nitrogen-compound contamination in sampled gas, an interference-calibrated detection system for detecting contaminant gases, an interference-calibrated method for measuring the concentration of basic nitrogen compounds in a target gas sample, a detection system for detecting a contaminant gas and a photolithography system as claimed in claims 1 and 9, 4 and 13, 6, 7, 11 and 14, respectively. Optional features are recited in the dependent claims.

The invention is based, in part, on the realization that semiconductor manufacturing and certain other processes, which are recognized to be sensitive to NMP, ammonia, or other basic nitrogen compounds, are in fact sensitive to the total proton-bonding capability of all nitrogenous base contaminants present, regardless of the specific identity of the nitrogenous base contaminants. According to the invention, rather than determining the presence and concentration of each individual contaminant by a separate detector, it is realized that important advantages can be obtained by providing a detector that provides a single reading that is stoichiometrically related to the aggregate proton-bonding characteristic of various nitrogenous base contaminants that may be present in the monitored air. In this way, a "total basic-nitrogen-compound detector" is provided.

As explained further, below, what is recognized to be of use is a measurement of the totality of those multiple basic-nitrogen-compound contaminants in the gas sample that can adversely affect the process being monitored. For instance, currently-employed DUV photolithography processes are sensitive to both strong and weak bases; hence, according to the present invention, all airborne basic nitrogen compounds are measured down to low concentration levels. In other cases, where the process is sensitive only to bases greater than a certain $pK_b$, the system is implemented, according to this aspect of the invention, to measure the totality of the multiple basic nitrogen compounds within the $pK_b$ range to which the process is sensitive, even at low-concentration levels.

The present invention focuses specifically on an apparatus and method in which a diagnostic gas of a known reference composition is passed through a detector that generates a reading of a nitrogen compound in the diagnostic gas. A control system may be used to determine how closely the detector reading corresponds to the reference concentration of a nitrogen compound in the diagnostic gas. A detected variance in the detector reading can then be used either as a basis for providing a calibration or to trigger an alarm, which can be in the form of a transmitted service-call request to provide notice of a system malfunction. Depending on its composition, the diagnostic gas can also be passed through a converter and, in some embodiments, through a scrubber as well. In other embodiments, two or more diagnostic gases of different compositions and concentrations of nitrogen compounds are passed through one or more detectors in an apparatus for detecting basic nitrogen compounds in a gas sample. Detector readings can then be compared to provide a span calibration or other forms of calibration to account for variances in the efficiency of scrubbers, converters or detectors.

A system for performing this method includes a sensor ($e.g.$, at least one detector), which operates as described above, as well as a primary channel that delivers a target gas sample to a detector and at least one scrubbing channel that delivers a reference gas sample to the same or a different detector. The system also includes at least one converter, which converts basic nitrogen compounds into an indicator gas, coupled to the primary channel and to the scrubbing channel. Further still, the system includes two or more gas sources coupled with the convertor apparatus and sensor. The gas sources each supply a gas with a distinct, known concentration of a nitrogen compound through the convertor and sensor. In preferred embodiments, the sensor includes at least two detectors, one coupled to the primary channel and another coupled to the scrubbing channel. In this embodiment, the converter apparatus includes a pair converters (one coupled to the primary channel and the other coupled to the scrubbing channel), thereby enabling simultaneous measurements to be made from both channels.

A control system can be included in the detection system. The control system may be programmed and configured to (a) generate commands to direct gas samples from each of the pair of gas sources through the sensor, (b) receive data values (or "readings") from the sensor for each gas source, (c) calculate a difference in data values, (d) generate a span calibration value, and (e) apply the span calibration value to subsequent data values for a process gas to generate a scaled value indicative of the basic-nitrogen-compound level for the process gas. Alternatively, or in addition, the control system is programmed and configured to compare data values with known reference concentrations of nitrogen compounds in a diagnostic gas source either to evaluate whether the detection system is functioning properly or to generate a calibration value for the detection system. The control system includes a processor, interfaces for receiving detector readings and a computer-readable medium storing software code for performing the above-described functions.

Other aspects of the invention focus on the basic-nitrogen-compound scrubber system. While a single ion exchange bed can be provided in a channel to remove ammonia from a gas sample flowing through the channel, this solution is not without drawbacks. Of particular concern is that ion exchange beds over time do not effectively filter basic nitrogen compounds other than ammonia. For example, NMP, a higher-molecular-weight imide, can pass through a 5 cm (2 inch)-deep, 1·3 cm (½ inch)-diameter scrubber in about 15 hours triggering false-negative signals in a measurement of total basic nitrogen compounds. This is a major problem which affects the functionality and reliability of the instrument's output.

In preferred embodiments of a detection system of this invention, a primary channel and a plurality of scrubbing channels are connected to a sensor. The term, "sensor" (or "detector apparatus"), as used herein includes a single detection device/unit as well as a plurality of detection devices/units. Each of the scrubbing channels includes a basic-nitrogen-compound scrubber. Downstream from each of the scrubbers is a converter which converts gaseous nitrogen compounds into an indicator gas and a detector for detecting the indicator gas. A primary channel also leads to the converter apparatus and sensor. As used, herein, the term, "converter apparatus," refers either to a single converter unit to which all channels are connected, in parallel, or to multiple converter units individually associated with a single or multiple channels.

In preferred embodiments of a method of this invention, reference gas samples are passed through first and second scrubbers to remove basic nitrogen compounds from the gas samples. The second scrubber is purged to remove reversibly-bound nitrogen compounds while a gas sample passes through the second scrubber. The gas samples passing through either the first or second scrubber then pass through the converter apparatus and sensor. Alternately, a target gas sample bypasses the scrubbers before delivery to the converter apparatus and sensor. The total concentration of basic nitrogen compounds in the gas samples is then determined on the basis of the difference of the detected concentration of the indicator gas in the target gas sample and the detected indicator gas concentration in the reference gas sample.

In further preferred embodiments of the system, the scrubbing channels are connected in parallel, and the system includes purge systems coupled to the scrubbers for purging reversibly-bound basic nitrogen compounds from the scrubbers. Preferably, the scrubbers include cation exchange media. A flow controller governed by a control system can be positioned to selectively control which of the scrubbing channels the sampled gas can flow through to the converter apparatus. The control system can be programmed to transfer the flow of the reference gas sample that reaches the sensor from a scrubbing channel with a contaminated scrubber to a scrubbing channel with a purged scrubber and then to direct a purge gas through the contaminated scrubber. Preferably, the control system is programmed to transfer the flow of the reference gas sample away from a scrubbing channel and to purge the scrubber of that scrubbing channel before a weak-base nitrogen compound can penetrate through the scrubber. Moreover, the purging process can alternate between scrubbers with the flow of gas samples to the converter preferably continuing substantially without interruption. Finally, the control system can be programmed to alternately transfer the flow of a gas sample between the primary channel and one of the scrubbing channels.

In accordance with one aspect of the invention, the system samples gas from a photolithography tool cluster for the fabrication of semiconductor wafers and is adapted to monitor the concentration of basic nitrogen compounds in the photolithography tool cluster. In a specific form of this embodiment, the scrubbers include photoresist-coated beads, the photoresist coating matching the photoresist applied by the photolithography tool cluster.

Other preferred embodiments of the system include a pressure reducer located upstream from the sensor and a vacuum pump located downstream from the sensor. Further, a common converter and detector can be coupled to the primary channel and the filtering channels. Further still, scrubbers are preferably excluded from the primary channel, and at least one multi-way valve is preferably arranged for selecting which of the channels the sampled gas may flow through to the converter.

Particular advantages of this invention include the ability to accurately and reliably determine whether the basic-nitrogen-compound detection system is functioning as expected and intended. By providing diagnostic gas sources and a control system for comparing known reference concentrations of a nitrogen compound in a diagnostic gas with detector readings, this invention is used to determine whether the detector(s) of the detection system is/are providing accurate readings of the concentration of nitrogen compounds in the sampled gas. If the detector readings do not match the known reference concentration of a nitrogen compound in a diagnostic gas, the control system can then automatically trigger an alarm, preferably transmitting a service-call request to a service provider to provide notice to remedy the malfunction. Further, the methods and apparatus of this invention can be used to generate calibration values that can be applied to subsequent detector readings to compensate for variances in the efficiency of a scrubber, converter or detector and thereby provide for more accurate and reliable readings from the system.

## BRIEF DESCRIPTION OF THE DRAWINGS

Fig. 1 is a plan view of a DUV photolithography processing facility employing a contaminant detection system.

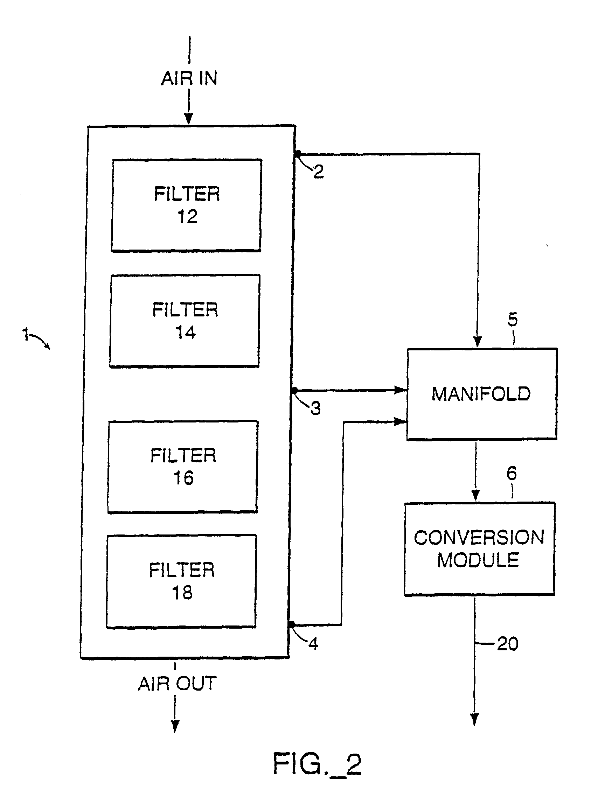

Fig. 2 is an enlarged schematic view of the filtration tower shown in Fig. 1.

Fig. 3 is a flow diagram illustrating the process of zero calibrating the detection system of Fig. 1.

Fig. 4 is a flow diagram illustrating the continuous operation and calibration of the embodiment of Fig. 1.

Fig. 5 is a flow diagram illustrating the monitoring and control of the processing tools and filtration system of the embodiment of Fig. 1.

Fig. 6 is a schematic view of a total basic-nitrogen-compound detector as a mobile detection unit.

Fig. 7 is a schematic view of a photolithographic system in which a total basic-nitrogen-compound detector is combined with a track.

Fig. 8A is a schematic view of a sample delivery train for total basic-nitrogen-compound detection that has a scrubber system to produce an internal reference, and in which a pressure reducer is located downstream from the scrubber system.

Fig. 8B illustrates a basic-nitrogen-compound scrubber system.

Fig. 8C illustrates a sample delivery train with separate pressure reducers.

Fig. 8D illustrates a thermal converter useful in the system of Fig. 8A.

Fig. 8E is a schematic view of a detection system that includes three separate sample channels coupled to an NO detector.

Fig. 9 is a schematic view of a sample delivery train similar to that of Fig. 8A in which an isolation valve is located upstream from a scrubber system.

Fig. 10 is a view similar to Fig. 8A of a system implemented to be immune to variations in NO and other interferents at the sampling site.

Fig. 11 is a schematic view of a sample delivery train similar to that illustrated in Fig. 8A and further including a zero source and a span source for performing a span calibration of this invention.

Fig. 12 is a flowchart illustrating a span-calibration process of this invention.

Fig. 13 is a flowchart illustrating a process of this invention wherein the span calibration, along with zero calibration and interference calibration, is applied to subsequent detector measurements.

Fig. 14 is a schematic view of a photolithographic apparatus including a diagnostic system.

Fig. 15 is an illustration of a computer-generated sample-location menu.

Fig. 16 is an illustration of a computer-generated toolbar.

Fig. 17 is an illustration of a computer-generated window for a data acquisition schedule.

Fig. 18 is an illustration of a computer-generated window for a sample locations display.

Fig. 19 is an illustration of a computer-generated window for a local alarm display.

Fig. 20 is an illustration of a computer-generated window for a real-time sampling monitor.

Fig. 21 is an illustration of a computer-generated window for a system of this invention including an ion mobility spectrometer.

## DESCRIPTION OF THE PREFERRED EMBODIMENTS

A description of preferred embodiments of the invention follows.

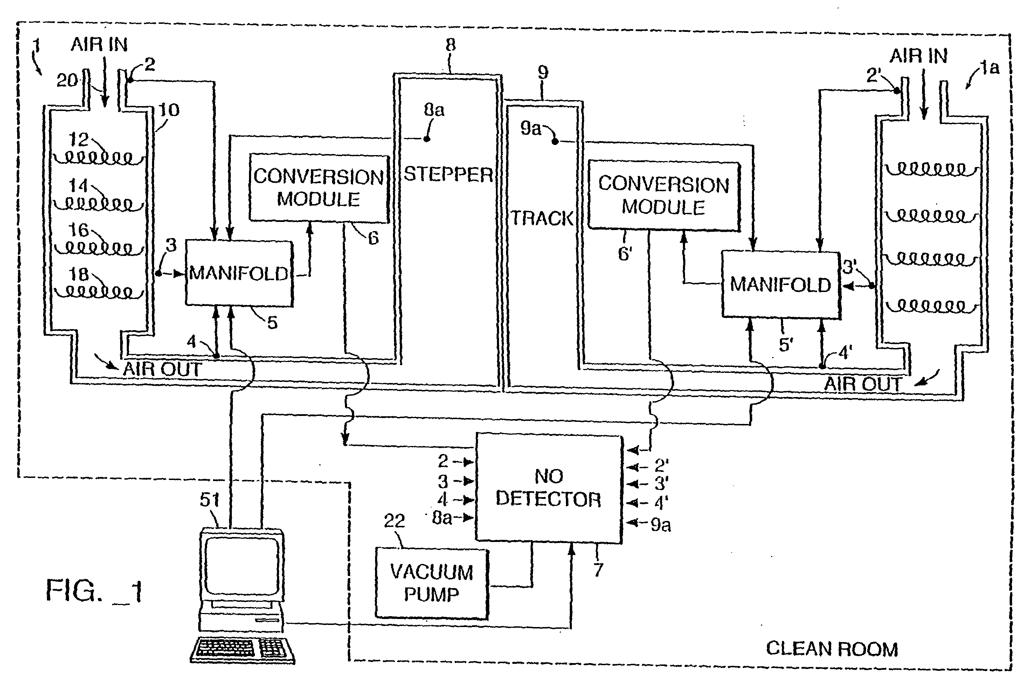

In Fig. 1, a photolithography tool cluster is shown for the production of semiconductor wafers. The cluster consists of two tools, a stepper 8 and a track 9. A wafer processed by the cluster is coated with photoresist in the track 9, transferred to the stepper 8 where the coated wafer is exposed to ultraviolet radiation passing through a reticle, and then transferred back to the track 9 where the exposed photoresist is developed. Each of these tools 8, 9 is joined to a separate clean air filtration system, 1 and 1a, respectively. Each filtration tower comprises a metal enclosure 10 and a set of spaced apart chemically-active filter stages 12,14,16,18 installed in series within the enclosure. As depicted in Fig. 1, the air enters at 20, at the top of the tower, the air being supplied from either outside the fabrication facility or from within the facility, or from within the clean room or the tool itself. This system and its operation are more fully described in US-A-6,096,267 and US-A-6,296,806.

The filters are composed of chemically-active composite materials, typically nonwoven fabric media, to which are bound activated carbon particles or ion exchange beads that have been treated to remove ammonia and organic amines. The filter media is typically arranged as a set of pleats in the enclosure. An example of such filter media is known by the trademark, Vaporsorb™, produced by the Assignee, Extraction Systems Inc. of Franklin, Massachusetts, U.S.A.

In another embodiment, a converter-detector apparatus is employed to monitor filtering performance of a filter deployed in either the make-up or recirculation air supplying a clean room. In this case, the converter-detector apparatus is employed in such a manner as to monitor total basic nitrogen compounds both upstream and downstream of a filter deployed either alone or in series in the make-up or recirculation air system of the clean room.

In other implementations, different filter media are employed. Certain examples include parallel trays of loose activated carbon particles produced by, *e.g.*, Donaldson Company (Minneapolis, Minnesota, USA); extruded carbon blocks using a dry thermoplastic adhesive as the binding agent as produced by, *e.g.*, Flanders Filters (Washington, North Carolina, USA), KX Industries, and Peneer Industries; thin extruded carbon blocks manifest as a fabric as manufacturing by, *e.g.*, KX Industries; media made by the modification of the chemical properties of the fiber structure as produced by, *e.g.*, Ebara Corp. (Tokyo, Japan) and Takuma Ltd.; and carbon fiber structures as produced by, *e.g.*, Kondoh Ltd.; and carbon particle sheet media produced by, *e.g.*, Hoechst-Celanese.

As shown in Fig. 1, each filtration tower, 1 and 1a, includes, respectively, an upstream sampling port 2, 2', a downstream sampling port 4, 4', and an intermediate sampling port 3, 3'. Sampling ports 8a and 9a are likewise provided for the stepper 8 and track 9, respectively. For each filter and tool combination, there is one conversion module 6 (for the stepper 8) and 6' (for the track 9). The conversion modules 6 and 6' are connected to a common, remotely-located NO detector 7.

In other embodiments, a single conversion module receives gas samples from both tools 8 and 9 and delivers the converted samples to the detector 7. In this case, the conversion module and the detector 7 can be in the form of a Model 17 instrument, which is available from Thermo Environmental Instruments Inc. (Franklin, Massachusetts, USA). Although the remaining description relating to Fig. 1 is generally directed to the illustrated embodiment, which includes a pair of conversion modules 6 and 6', a single conversion module can generally be used with equal success.

A remotely-controlled manifold 5, 5', is associated with each conversion module 6, 6', respectively. Via respective sample lines, the manifolds 5, 5' direct to the conversion modules 6, 6' respective samples from the tools 8, 9, from the inlet streams 2, 2' of the filters, from the outlet streams 4, 4' of the filters, and from the intermediate filter ports 3, 3', according to a sequence controlled by a computer 51. As shown in Fig. 8A, and as described in greater detail, below, the conversion modules 6, 6' include a pair of channels, A and B, through which the flow of sampled gas is alternated every 10 seconds, a scrubber system 120 in channel A, a multi-way valve 122 for controlling and redirecting flow through channels A and B, a pressure reducer 118, and a thermal catalytic converter 124 in which nitrogen compounds in a gas sample are converted to nitric oxide (NO).

The converted samples are drawn from the conversion modules 6, 6' to a detector 7 by a vacuum pump 22 located downstream of the detector 7. The detector 7 includes a reaction chamber where NO is oxidized to produce electronically-excited $NO_2$ molecules and a photomultiplier tube which measures photons emitted by the electronically-excited $NO_2$ molecules as they return to their ground state.

In one embodiment, an impinger is employed to identify possible contaminants in the unconverted gas sample. The impinger consists of a glass or quartz tube holding a liquid. In this embodiment, a vacuum pump and an associated calibrated flow controller are employed to draw the gas sample from one of the sample lines through the liquid to take a grab sample. The grab sample is then analyzed or subjected to real-time colorimetric analysis, providing a quantitative assessment of the contaminating basic nitrogen compounds in the sampled gas.

In an instrument associated with more than one conversion module, each converter-detector subsystem is considered as a single instrument, which is calibrated independently of the other converter-detector subsystems. In the preferred embodiment illustrated in Fig. 1, there are two converter-detector subsystems: one subsystem serves the track 9 and its air filter system 1 and the other subsystem serves the stepper 8 and its air filter system 1a. To perform a "zero" calibration, zero air (*i.e.*, air free of basic nitrogen compounds) is provided by filter system 1 for the stepper subsystem and by filter system 1a for the track subsystem. By having the conversion module 6, 6' near the sampling area, the length of the sampling lines exposed to basic nitrogen compounds is reduced, which increases the response time of the system.

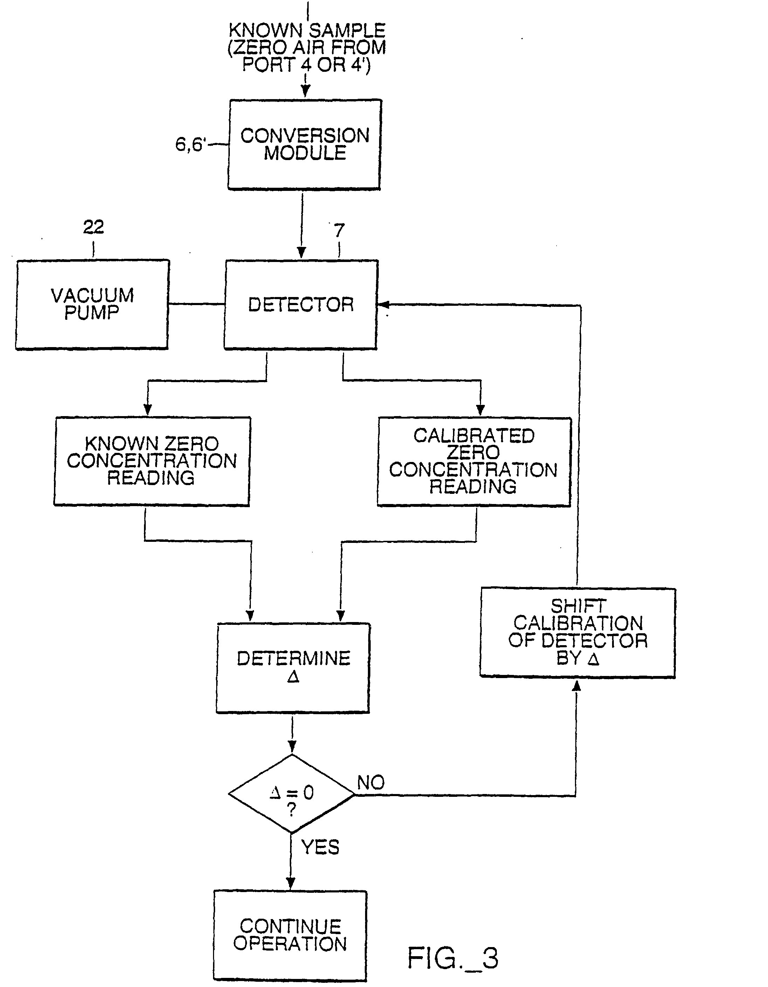

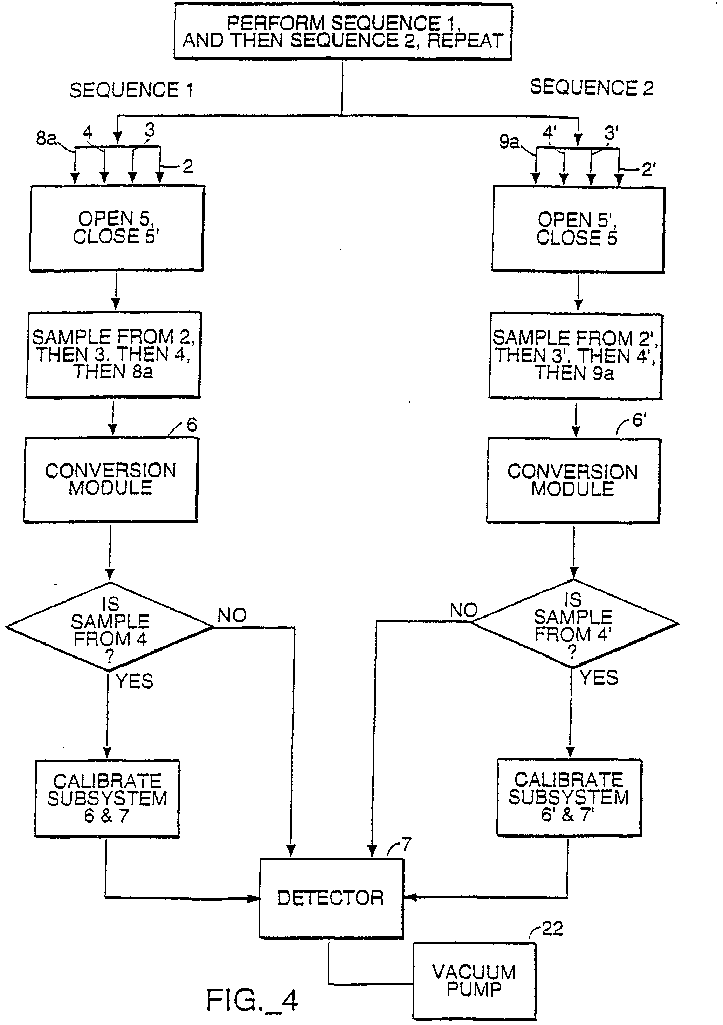

To zero calibrate each converter-detector subsystem, two or more samples of known reference concentration of contaminants are provided to the instrument, as illustrated in Fig. 3. The instrument response is then compared with the known reference concentrations, and a zero calibration curve is generated and associated either manually or electronically (through software) associated with the instrument to provide corrections to the instrument's response. In general, the instrument response over the concentration range remains stable for an extended period. The instrument is sensitive, however, to loss of zero calibration, for reasons such as drift of the photomultiplier, and the curve must be shifted relative to the true zero reading as it varies over time. Because harmful contaminant concentrations in photolithography processing are extremely low (on the order of 1 part per billion (ppb) or lower), zero calibration in preferred systems is typically performed on the order of once a month to assure the fidelity of the zero reading. In a particularly preferred implementation, the detection system is arranged to operate continuously, as shown in Fig. 4, whereby the system performs a total basic-nitrogen-compound detection for each of the sampling ports in turn and conducts two zero calibrations each cycle, one with respect to each of the conversion modules 6, 6' with which the detector 7 operates.

Preferably, the zero air employed for calibration is provided by the outlet ports 4 and 4' of the filtration system (see Fig.1). The instrument is then instructed to provide a zero reading for the calibration sample. In the case that the difference between the total basic-nitrogen-compound reading for the outlet port 4 and the sample at the intermediate port 3 is not greater than zero, the sample from the outlet port 4 is employed to establish zero air. In another preferred embodiment, a sampling port located just preceding the last filter stage is employed to verify that the zero air from the output of the filter stack is in fact zero air. Also, in an alternative embodiment, a built-in, dedicated, zero air generator is employed. The generator provides zero air by either filtering the ambient air or by bubbling air through a liquid scrubber solution.

An external computer, preferably situated outside the clean room in which the tools are located, is employed to control the operation and monitor the entire photolithography process. The software is customized for the intended application. Performance data is provided to the computer to provide an archival database to be employed to give the contamination history of the tool cluster.

Based on the particular ports being sampled, the software employed in the operation of the instrument determines which converter-detector subsystem is to be calibrated and the appropriate source of zero air for calibration purposes. The software also designates which calibration curve to employ. As the detection system is calibrated and the new zero readings are determined, the calibration curves are adjusted accordingly.

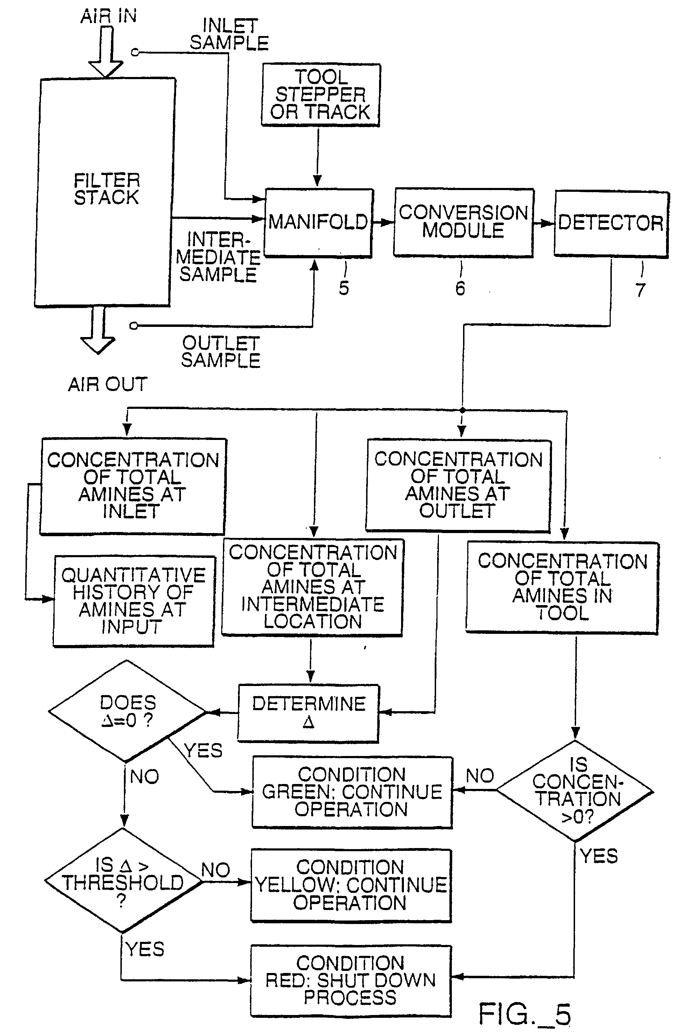

In a desired application, control instrumentation, as illustrated in Fig. 5, monitors the performance of the filtering system and the level of contamination at the track and stepper tools. Should a reading from either the stepper or track exceed a predetermined threshold, an alarm is activated and the process is immediately shut down. However, by use of this detection system, the occurrence of such an emergency can normally be avoided.

As shown in Fig. 5, the filtering system is continuously monitored in real time, as follows. The sample at the inlet to the filter system, over time, provides a quantitative history of the input of basic-nitrogen-compound contaminants to the filter. By using samples drawn from the intermediate position along the filter system as well as from the outlet of the filter stack, and by measuring the difference in concentration levels from these locations, one of the following steps is triggered. If the difference is zero (condition green) and the total amine or Bronstead base concentration at the tool is within operating limits, then the operation continues without interruption. When the difference is greater than zero, the difference is compared with a predetermined threshold. If the threshold is not exceeded (condition yellow), operation continues but a filter replacement is scheduled. If the threshold is exceeded, or if the total basic nitrogen compound detected at the tool exceeds operating limits (condition red), the operation is immediately shut down.

In another embodiment, there are three or more conversion modules remotely located at various locations in the fabrication facility. One conversion module is employed to monitor the general conditions in the clean room; a pair of conversion modules is employed to monitor the contamination around a different tool cluster; another conversion module is employed to monitor the contamination level within a chemical storage cabinet to provide early indication of chemical spills. A central controller is used to monitor detector readings associated with each of these conversion modules. Accordingly, the central controller can identify a particular line in the fabrication facility where basic-nitrogen-compound contamination is a problem and can sound an alarm identifying the problematic line so that remedial action (*e.g.*, filter replacement) can be taken.

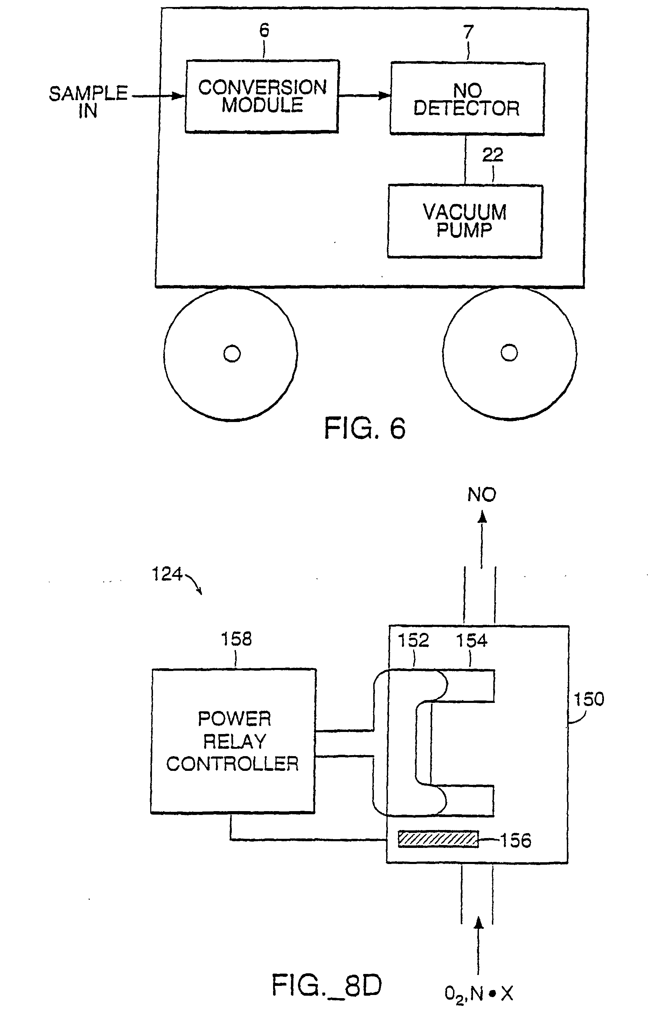

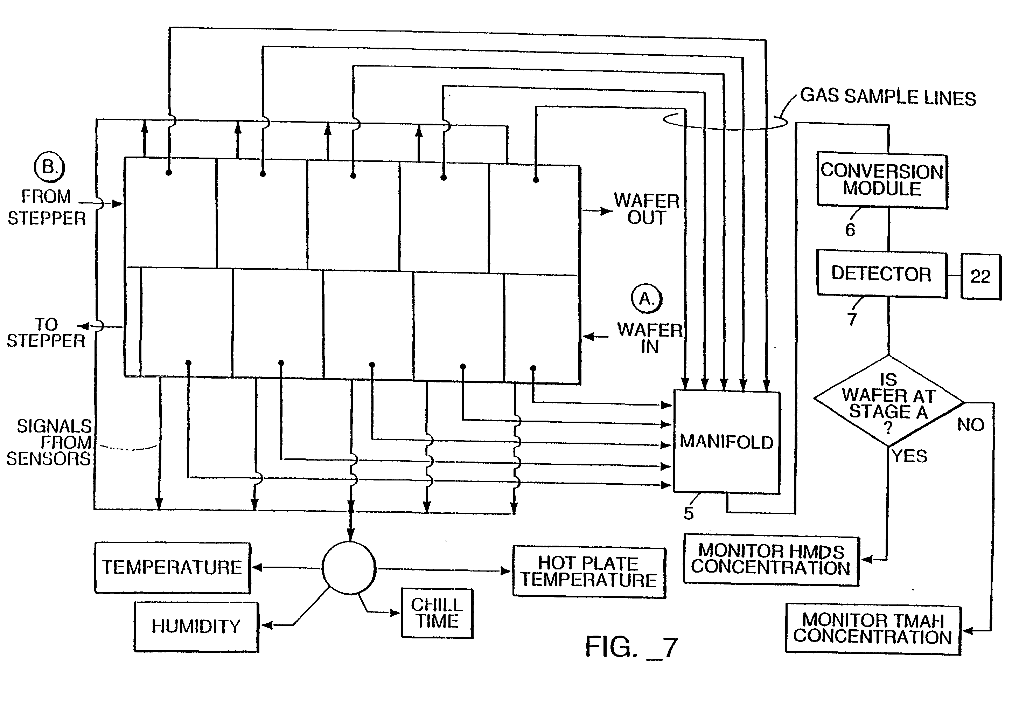

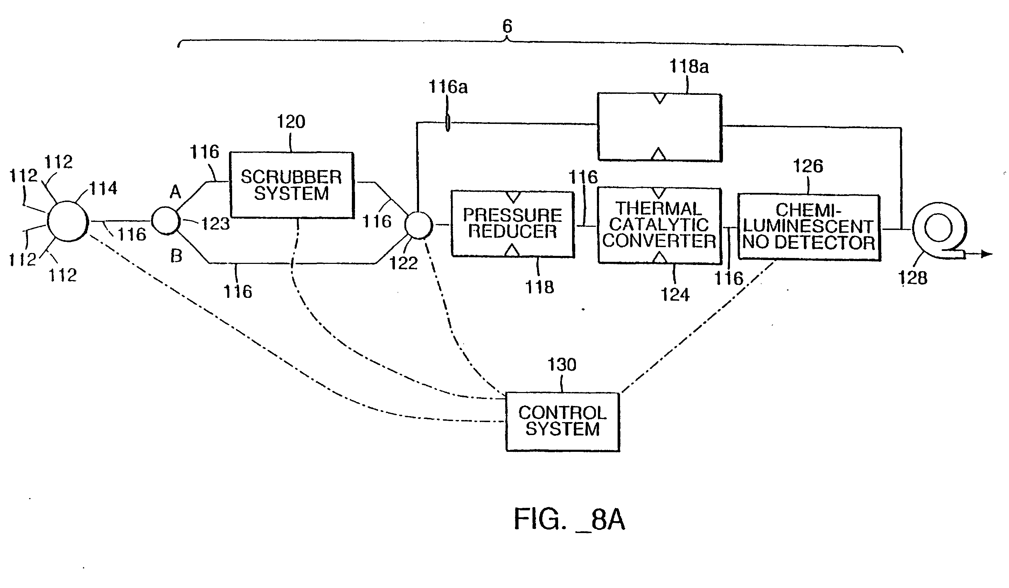

In another implementation, shown in Fig. 6, the converter-detector instrument is constructed as a mobile leak detector. The mobile unit is moved to selected regions of the fabrication facility to seek possible areas of contamination leaks. By following an escalating basic-nitrogen-compound concentration trend, the mobile unit localizes the source of the contamination.

In another embodiment, illustrated in Fig. 7, the system is combined with a multi-point sampling system of an array of sensors to monitor the operating status of a track, including temperature, temperature of the hot plate, time on the chill plate, exposure time, etc. A total basic-nitrogen-compound detector monitors process contaminants, such as the concentration of an adhesion promoter, *e.g.*, hexamethyldisilozane (HMDS), in a gas sample during the coating stage when photoresist is applied to the semiconductor wafers. The wafers are then sent to the stepper for exposure and subsequently brought back to the track for developing. During this stage, another, or the same, total basic-nitrogen-compound detector monitors the concentration of another possible internally-processed chemical contaminant, such as tetremethylammonium hydroxide (TMAH), employed in the developing stage.

The system enables, in its total basic-nitrogen-compound reading, the simultaneous detection of NMP and ammonia, which previously were typically monitored with separate detectors. The system also enables detection, in its total basic-nitrogen-compound reading of other basic nitrogen compounds that are known to be harmful to the photolithography process, such as morpholine, diethylamine ethanol, and cyclohexylamine, agents which are commonly used to inhibit corrosion in high-humidity regions. Basic nitrogen compounds from the facility cafeteria, especially seafood, are also included in the detection as well as basic nitrogen compounds from the breath of the facility workers, which can create high levels of basic-nitrogen-compound contamination, depending upon diet and smoking habits. As has been explained, the system, as illustrated, converts substantially all such airborne basic nitrogen compounds to a common detectable compound, which it detects to indicate the level of hydrogen-bonding contaminants. If high concentrations of the contaminants are detected, by grab sampling techniques, the exact sources of the contamination can be determined and remedied.

Another advantageous aspect of the system is its adaption to the certification process of clean rooms. Heretofore, during the certification process, each individual molecular base present in the clean room was typically detected by a separate detector. The concentrations were summed providing a number indicating the total base loading in the clean room. For instance, if three bases were present, each with a concentration of 10,000 parts per trillion (ppt), the clean room rating would be MB30,000 (or 30,000 ppt). the present invention solves the problem of detecting individual bases by providing the total base loading within a clean room with a single reading.

The following embodiments of the invention are particularly effective in providing an accurate determination of total basic nitrogen compounds at low concentrations, for protection of the base-sensitive processes. These embodiments include a primary channel and a plurality of scrubbing channels for delivering gas samples from a source to the converter and detector. Each of the scrubbing channels includes a removal device for removing basic nitrogen compounds from the gas samples to produce reference samples. In some embodiments, the removal device is process-specific, meaning that it removes only those basic compounds that are of interest for a specific process. For example, in an embodiment specifically designed for use with semiconductor fabrication tools, the removal device is coated with photoresist to remove from the gas sample those compounds that will interfere with photolithographic deposition and development. The primary channel, meanwhile, delivers target gas samples from the same source to the convertor without scrubbing.

Because the scrubbing channels produce a reference sample that is free of substantially all of the gas molecules corresponding to the class of basic nitrogen compounds of interest, the reference sample provides a baseline for canceling the effects of background, nitrogen-containing contaminants which might otherwise contribute to the NO concentration detected by the chemiluminescent detector in the target gas sample. The baseline reading is then subtracted from a reading performed on a target gas sample.

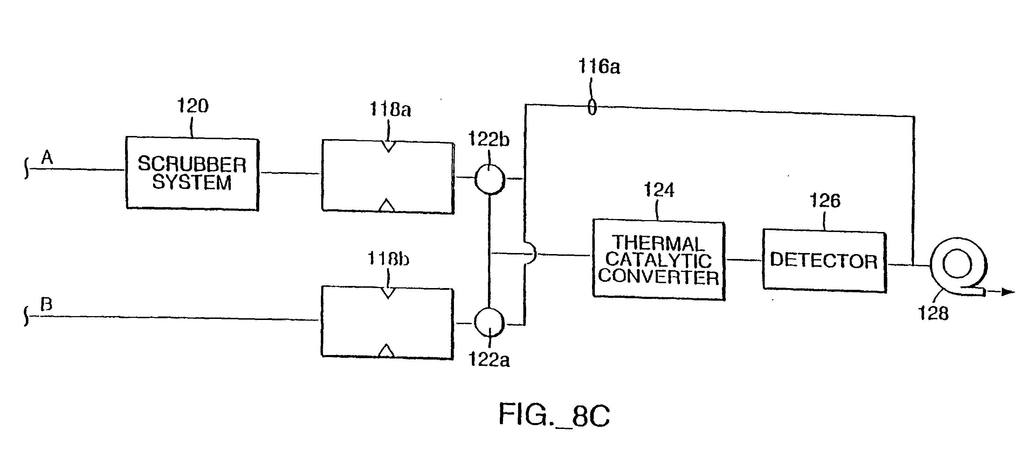

Referring to Fig. 8A, a sample delivery train, including a conversion module 6, is shown for determining the total basic-nitrogen-compound concentration in a gas sample using an internal reference for zero air (i.e., air that is free of basic nitrogen compounds). The sample enters through a selection valve 114 (e.g., a multi-position valve manufactured by Vici Co., Houston, Texas, USA), which creates an open flow path from one of the sampling ports 112. From the selection valve 114, the sample is directed to the conversion module 6, which splits each incoming sample into two parts, channels A and B, and directs each part separately, via a multi-way valve 122, to a thermal catalytic converter 124 followed by a chemiluminescent detector 126. Alternatively, a pair of three-way valves (illustrated as valves 122a and 122b in Fig. 8C and connected as shown) can, in practice, be substituted for the multi-way valve 122. Another valve 123 is provided upstream from the scrubber system 120 to help direct the flow of the gas sample into channels A and B and to prevent backflow through the channels.

Channel A, which serves as a "scrubbing channel," directs part of the sample through a basic-nitrogen-compound scrubber system 120 and then to the valve 122, while channel B, referred to as the "primary channel," directs part of the sample directly to valve 122. The term, "scrubber," as used in this document is intended to refer to any device that is effective for removing basic nitrogen compounds, or a device that otherwise treats the basic nitrogen compounds in such a way that they have no effect on the response of the detection system being employed.

The scrubbed and unscrubbed parts of the original sample from channel A and B, respectively, proceed from valve 122 through the thermal catalytic converter 124 to the chemiluminescent detector 126 as time-separated samples in a single conduit. The difference between the response of the chemiluminescent detector 126 to samples from channel A and B reflects the total basic-nitrogen-compound concentration in the original sample of gas.

It has been determined that the sensitivity of basic-nitrogen-compound detection improves as the operating pressure of the chemiluminescent NO concentration measurement decreases. Accordingly, the chemiluminescent NO detector 126 operates at a pressure of 125 millibar or less, by co-action of vacuum pump 128 and an upstream pressure reducer 118, to achieve a low noise level and to achieve a detection sensitivity of 1 part per billion (ppb), preferably 0.5 ppb, or better. Though the ideal operating pressure in the detector 126 is a function of the gas flow rate through the detector in any given application, a preferred pressure level in the detector is generally about 40 mbar (about 30 Torr).

The conversion module 6 initially takes a sample of gas from one of the sampling ports 112 via selection valve 114 and channels it through one of the sample lines 116. Sample lines 116 are tubes formed of Teflon® PFA from E.I. DuPont de Nemours of Wilmington, Delaware, USA. Alternatively, sample lines 116 can be stainless steel tubes coated with CVD-coated silica (e. g., Silcosteel™ from Restek Corp., Bellefonte, Pennsylvania, USA). Sample lines coated with silica are nonporous and nonreactive and, therefore, have little effect on ammonia and organic amines passing through the tubes. In contrast, commonly-used tubing of PTFE is relatively porous and tends to emit hydrogen fluoride, a strong acid, which reacts with ammonia and amines, interfering with the measurement of total basic nitrogen compounds. Silica may be deposited onto the stainless steel tubing using chemical vapor deposition. In certain applications, sample lines comprising glass are used. The glass sampling lines may be reinforced with epoxy.

In certain advantageous embodiments, sample lines 116 are heated substantially along their total length to approximately 50° C using electrical heating lines (see, e.g., U.S. Patent 3,727,029). This reduces the tendency for basic nitrogen compounds to deposit on the walls of the tubing (reduces basic-nitrogen-compound-sticking coefficient) and thus reduces sample line contamination of the alternating sample gas slugs.

In this context, the combination of heating the sample lines along with using a silica coating on the lines is advantageous because it reduces both contamination of the gas sample and deposition of gas sample components. Nevertheless, the practice of heating sample lines of other compositions, by itself, can provide a beneficial effect. Heating is preferably accomplished using electrical resistance wire incorporated in the wall of the sample line or otherwise disposed in thermal contact with it.

Selection valve 114 (e.g., a multi-position valve manufactured by Vici) enables samples from different locations to be channeled into a single conversion module 6. The gas sample then flows through channel A or B to multi-way valve 122. After exiting valve 122, the gas sample passes through a pressure reducer 118 (e.g., a flow restrictor, such as a capillary glass tube or a small orifice), where the pressure drops from atmospheric pressure on the upstream side of pressure reducer 118 to approximately one-tenth atmospheric pressure on the downstream side. This pressure drop is maintained by vacuum pump 128 positioned downstream of detector 126. The pressure reducer 118 is preferably positioned downstream of the scrubber system 120 because the scrubber system 120 operates with greater effectiveness at higher pressures. Preferably, the pressure reducer 118 comprises a calibrated glass capillary heated to 50° C to reduce the basic-nitrogen-compound sticking coefficient. The pressure reducer 118 can be made from tubes of glass, ceramic, stainless steel, quartz, or stainless steel with an interior plated with gold or other inert material. The low pressure of the samples- created by pressure reducer 118 and vacuum pump 128, in addition to enabling high sensitivity detection, reduces the response time for measuring total basic-nitrogen-compound concentration. This is because the samples travel through the delivery train rapidly; for instance, in the system described, valve 122 may be shifted between channels A and B every 10 seconds in normal operation.

Auxiliary conduit 116a is connected at one end to valve 122, At its opposite end, auxiliary conduit 116a is connected to the vacuum pump 128. Auxiliary conduit 116a includes a pressure reducer 118a to limit flow there through. During operation, valve 122 connects the non-selected channel A or B to the auxiliary conduit 116a to maintain a gas flow through the non-selected channel. In this way substantially-steady-state flow conditions can be maintained in the scrubber system 120, and fresh sample is immediately available to the converter 124 upon actuation of valve 122. Further, the steady-state flow of gas through the channels reduces the incidence and magnitude of pressure spikes that can occur upon switching and thereby produce erratic instrument readings.

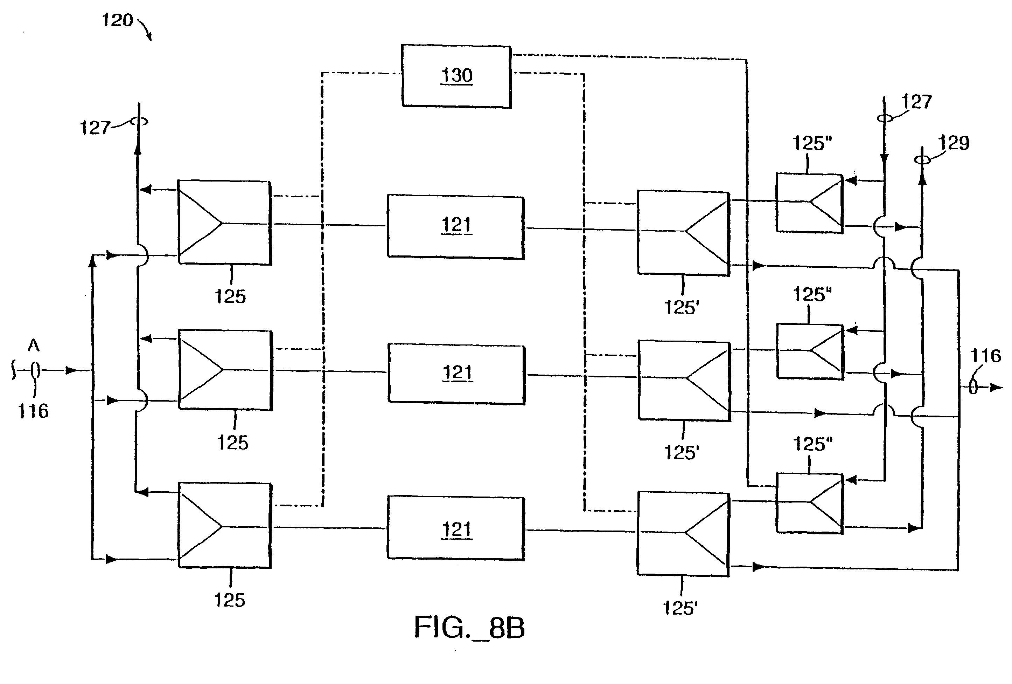

The components of a scrubber system 120 of this invention are illustrated in Fig. 8B. The scrubber system 120 includes three scrubbers 121 connected in parallel to the sample line 116. The scrubbers 121 of channel A are constructed to selectively remove from the gas sample the totality of the basic nitrogen compounds to which a photolithographic or other process being guarded is sensitive, yet their construction and function are nevertheless such as to not affect other nitrogen-containing compounds. Each scrubber 121 is preferably a solid-state scrubber, comprising an ion exchange medium with active sulfonic or carboxylic groups. The ion exchange medium is substantially non-hygroscopic and nonporous. The medium can be in the form, for example, of either fibers or a resin. The scrubber may also comprise any material that preferentially binds the airborne molecular bases (e.g., photoresist-coated substrates, weak acid-coated substrates, strong acid-coated substrates, ion exchange materials, or chemically-treated activated carbon and molecular sieves). The properties of the active media can be chosen to optimize the selectivity of the detection process. Further still, the scrubber can be a chemical gas filter medium, along the lines of the air filter that supplies "zero air" in the previously described embodiments.

A strong cation exchange medium is preferred as the scrubber substance in the scrubbers 121 of Fig. 8B. The medium within basic-nitrogen-compound scrubber 121, being a strong acid, removes multiple molecular bases, such as ammonia and other basic nitrogen compounds. A strong cation exchange medium ensures removal of both strong and weak molecular bases, and is suitable for photoresist lithography techniques that are sensitive to both strong and weak bases. For techniques sensitive only to strong bases, a weaker acid ion exchange medium may be employed, so that weak bases are not removed and occur equally in the scrubbed and unscrubbed samples.

Nevertheless, even when a strong cation exchange medium is used, chromatic differences in removal efficiency will develop over time. A strong base, such as ammonia ($NH_3$), will be tightly bound to the cation exchange medium and, once it is removed from the gas sample, will likely remain fixed to the scrubber. A weak base (typically, one with a high molecular weight, such as NMP), however, will tend to gradually drift through the scrubber, repeatedly binding to and releasing from the cation exchange medium. Gradually, over a matter of hours, NMP will work its way through and out of the scrubber. When a tube that is 1·3 cm (½ inch) in diameter and 61 cm (2 feet) in length is used with a flow rate of 1 liter per hour, NMP will pass through the scrubber in about 15 hours. Bases with strengths between that of ammonia and NMP will gradually drift through the scrubber, albeit at rates slower than that of NMP.

Nine three-way valves 125, 125' and 125" act as flow controllers, controlling the flow of sampled gas and purge gas through the scrubbers 121. The valves 125, 125', 125" in turn, are controlled by a control system 130. When a pair of valves 125, 125' on opposite sides of the same scrubber 121 both open channels to the sample line 116, sampled gas flows through that scrubber 121, with the scrubber 121 filtering basic nitrogen compounds from the sampled gas.

In one embodiment, the control system 130 selectively opens the channels to the sample line 116 in a single pair of opposing valves 125, 125' at a given moment. The control system then cyclically redirects the flow from the sample line 116 to each of the scrubbers 121, in sequence. The duration of flow (which is still received as a series of pulses, alternating with channel B, shown in Fig. 8A) through each scrubber 121 is limited to less than the time required for a high-molecular-weight contaminant to pass through the scrubber 121. Preferably, the gas flow is shifted among scrubbers 121 about every 2 to 3 hours. After the flow has been redirected from a scrubber 121, the control system 130 then sends a signal to respective valves 125, 125' and 125" to open a channel from the purge line 127 through the respective scrubber 121 and out the purge line exhaust 127', illustrated on the left side of Fig. 8B. The purge line 127 is supplied by a source of compressed purge gas (typically, air from which bases have been filtered), which flows through valves 125" and 125' and then to scrubber 121, so that the purge gas flows through the scrubber 121 in a direction opposite to the prior flow of the sampled gas. The purge gas is directed through each scrubber 121 via its respective valves 125, 125' and 125" in a sequence following that of the sampled gas flow and also for a period of about 2 to 3 hours. Accordingly, the purge gas cleanses each scrubber 121 of basic nitrogen compounds removed from the sampled gas. Consequently, a clean scrubber 121 is always available when the flow of sampled gas is redirected.

In addition to being connected to valves 125' and to the purge line 127, valves 125" are also connected to an exhaust line 129. In one embodiment, the control system 130 regulates valves 125, 125' and 125" to maintain a flow of sampled gas through each of the scrubbers 121 at all times, except during purging. The flow through only one of these scrubbers 121 is directed back to the sample line 116, however. Sampled gas flowing through other, inactive scrubbers 121 is directed by the valves 125' and 125" into the exhaust line 129, which removes the sampled gas from the system. The purpose of maintaining gas flow through "inactive" scrubbers is to keep "inactive" channels conditioned so that when the gas flow is redirected to a purged scrubber 121, the detector readings are less likely to be skewed by a momentary spike due to the need to flush old gas from the newly-activated scrubber 121 after a shift.

The sample in channel A, upon exiting scrubber system 120, contains essentially no detrimental molecular bases; *i.e.*, total concentration of objectionable basic nitrogen compounds is approximately zero. This basic-nitrogen-compound-free sample becomes the reference sample for purposes of measuring the total basic-nitrogen-compound concentration in the unscrubbed sample from channel B. The scrubbers, however, do not affect neutral or acidic nitrogen-containing compounds. Otherwise, a false signal would be produced because those same compounds remain in the target sample.

In other embodiments, the reference gas and the target gas may be sampled from different inputs. In these embodiments, it is desirable to construct channels A and B to have the same pressure drop from the input location to the converter input. Referring to Fig. 8C, separate pressure reducers 118a, 118b may be used in channels A and B. To ensure that the pressure drop in each channel is approximately the same, pressure reducer 118a in channel A contains a larger orifice than pressure reducer 118b to compensate for the affect of basic-nitrogen-compound scrubber system 120 on the pressure in channel A. Thus, the pressure drop between scrubber system 120 and three-way valve 122a is equivalent to the pressure drop between pressure reducer 118b and three-way valve 122b.

Valves 122a and 122b allow the basic-nitrogen-compound-free sample from channel A and the unscrubbed sample from channel B to be directed to thermal catalytic converter 124 alternately, in rapid sequence. Operating the delivery train at 125 millibar pressure, for example, enables the three-way valves 122a and 122b to jointly switch the flow of sample gas between channel A and channel B several times per minute, to enable averaging of a number of readings, if desired, within a short monitoring interval, for instance ten minutes or less. While either valve 122a or valve 122b directs a gas sample from one channel through the thermal catalytic converter 124 and detector 126, the other valve 122a or 122b maintains continuous flow through the other channel by directing gas that passes there through into auxiliary conduit 116a.

Thermal catalytic converter 124 (*e.g.*, as manufactured by Thermo Environmental Instruments Inc.) converts basic nitrogen compounds in each gas sample to nitric oxide (NO) by thermal oxidation. A suitable catalytic converter 124 is illustrated in Fig. 8D. The converter 124 comprises a reaction chamber 150 that may or may not contain a catalytic element 152 (*e.g.*, platinum and/or palladium), a heating element 154 to heat the reaction chamber, and a thermocouple 156 connected to power control relay 158 which regulates the temperature of the reaction chamber. Since any given sample from channel B may contain a variety of basic nitrogen compounds (such as morpholine, diethylamino ethanol, ammonia, and normal methyl pyrrolidinone), thermal catalytic converter 124 must have a high conversion efficiency for many types of basic nitrogen compounds. To achieve a high conversion efficiency (85-100%) for a broad range of basic nitrogen compounds, a stainless steel surface heated to 900°C is used as the catalyst. Alternatively, a metal oxide surface can be used. The gas sample is oxidized as it passes over the heated surface resulting in the conversion of basic nitrogen compounds to NO. If the gas sample lacks oxygen for oxidation, oxygen gas should be supplied to the converter.

In other embodiments, the converter may perform photocatalysis, wherein the bonds of nitrogen-containing molecules are split with ultraviolet light to free nitrogen atoms for oxidation to thereby form NO. The appropriate conversion technique is determined by the desired application, taking into account cost and conditions of use.

In some applications, gas samples entering thermal catalytic converter 124 may contain molecules that are not basic nitrogen compounds, but will nonetheless be converted into NO. For example, compounds such as $NF_3$, HCN and $CH_3CN$ are not basic nitrogen compounds, yet if present in the gas sample, will be converted to NO in thermal catalytic converter 124. These compounds will not affect the total basic-nitrogen-compound concentration calculation, however, because the basic-nitrogen-compound scrubbers 121 do not retain these compounds since they are not bases. As such, samples from channel A and channel B contain equal amounts of these non-basic compounds, and thermal catalytic converter 124 converts these compounds to NO equally for channel A and channel B, thus canceling out any effect.

Likewise, where the process to be monitored is sensitive only to strong bases, scrubbers that include a medium, *e.g.*, a weak acid, that removes only the various strong-base nitrogen compounds are selected. In this case, since the weak-base nitrogen compounds will be present in both channels, their presence does not affect the response of the system. Alternatively, basic nitrogen compounds can be differentiated on the basis of strength by using converters that convert bases of different strengths with different efficiencies.

Converted samples exit thermal catalytic converter 124 and enter chemiluminescence detector 126 (*e.g.*, as manufactured by Thermo Environmental Instruments, Inc.). The detector 126 employs chemiluminescence for NO detection. Typically, the maximum signal from chemiluminescence detector 126 is achieved at a pressure of about 65 Torr and at a flow rate of about 1.5 liters per minute under the operating conditions described above. Detector 126 operates at approximately 125 millibar, suitable for detection of low concentrations of basic nitrogen compounds. This high sensitivity affords detection of total basic-nitrogen-compound concentrations of less than 1 ppb, preferably less than 0.5 ppb.

Detector 126 employs chemiluminescence for NO detection. For this

$$NO + O_3 \rightarrow NO_2^* + O_2 \, ,$$

purpose NO is caused to react with ozone generated by an internal ozone generator in a reaction chamber of the detector 126. This produces electronically-excited $NO_2$ molecules ($NO_2^*$), which, in returning to the ground state, emit photons, $h\nu$, that are detected by an appropriately-cooled photomultiplier tube. The reaction is given by the expression,

$$NO_2^* \rightarrow NO_2 + h\nu.$$

To achieve the needed sensitivity for current DUV photolithographic processes with presently-available photomultipliers, the photomultiplier tube is cooled a temperature of -5° C or less. To achieve sensitivities required for next-generation fine-resolution DUV photolithography in semiconductor manufacturing, the tube is cooled to -15° C by an associated thermoelectric cooler. Moreover, since there is significant variation in the sensitivity of photomultiplier tubes produced by the same manufacture, the analyzer sensitivity is further increased by testing and choosing an optimum photomultiplier tube for the performance required. In other implementations, NO is detected by colorimetric methods using devices available from, *e.g.*, Tytronics, Inc. of Bedford, Massachusetts; other methods that are based upon continuous in-line sampling may also be used.

The signal from the photomultiplier is converted into time-based NO concentration values by a control system 130, and then the total basic-nitrogen-compound concentration of the gas sample from the selected sampling point is determined, *e.g.*, by appropriately averaging and differencing the values. The total basic-nitrogen-compound concentration for the gas sample equals or is proportional to (accounting for incomplete NO conversion of basic nitrogen compounds) the difference between the NO concentration of the unscrubbed sample from channel B, *e.g.*, at time $t_1$ (or the average of NO concentrations at times $t_1$, $t_3$, ... $t_{n-1}$, n being an even number) and the NO concentration for the basic-nitrogen-compound-free sample from channel A, *e.g.*, at time $t_2$ (or the average of NO concentrations determined at times $t_2$, $t_4$, ... $t_n$, n being an even number).

In an alternative embodiment, the detector 126 comprises a plurality of detection devices, with a separate detection device being allocated to each channel.

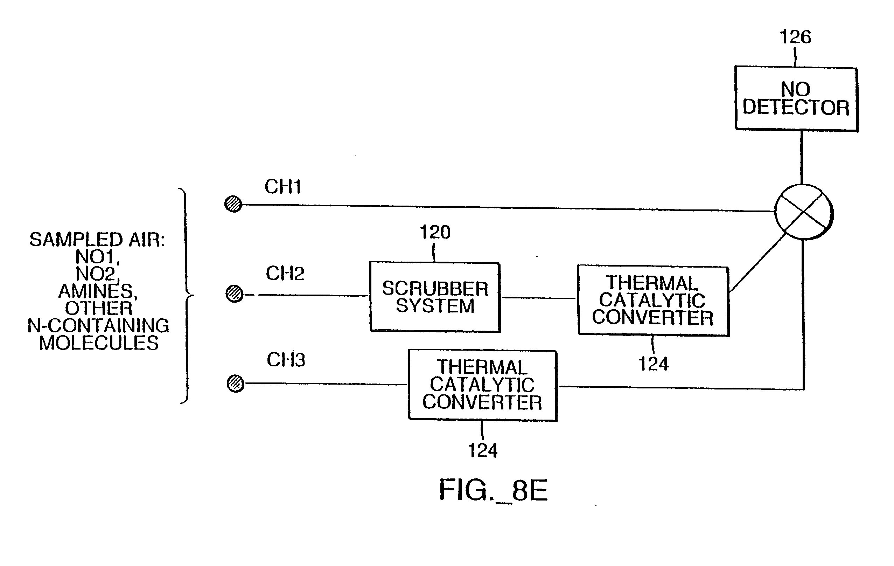

Additional channels may be used to provide further analysis of the components of the sampled gas. For example, referring to Fig. 8E, three channels (CH1, CH2 and CH3) may be used to determine the component concentrations of NO, $NO_2$ and other non-basic nitrogen-containing compounds which are convertible to NO, and the total basic nitrogen compounds in a gas sample. The following discussion focuses on the various classes of nitrogen-containing compounds in the sampled gas. NO, $NO_2$, basic nitrogen compounds, and other N-containing compounds reach the NO detector through CH1; and the NO detector produces a signal representative of the NO concentration in the sampled gas. NO (including NO converted from $NO_2$ and other convertible N-containing molecules) and non-convertible N-containing molecules reach the NO detector through CH2; and the difference between the detector signals for CH1 and CH2 provides a measure of the concentration of $NO_2$ and other non-basic, nitrogen-containing compounds which are convertible to NO in the sampled gas. NO (including NO converted from $NO_2$, basic nitrogen compounds and other convertible N-containing molecules) and non-convertible N-containing molecules reach the NO detector through CH3; and a measurement of the concentration of total basic-nitrogen-compounds in the sampled gas is obtained by subtracting the detector reading for CH2 from the detector reading for CH3. Accordingly, an interference calibration is provided by the above-recited steps.

In some implementations, variations that occur in the ambient NO and $NO_2$ concentrations may affect the accuracy of the total basic-nitrogen-compound concentration measurement. In such cases where the disturbance warrants, an algorithm is used to minimize the affects of such fluctuations by calculating the total basic-nitrogen-compound concentration based on a moving average for NO concentrations from channel A and channel B. For example, each NO concentration measurement is added to the previous consecutive measurements and divided by the total number of measurements made at that point in time. This moving average calculation may be represented by the following algorithm:

$$\text{Moving NO Average} = (X_1 + X_2 + ... + X_n)/n,$$

where X equals the NO concentration at a given time and n equals the total number of NO measurements made. The moving average calculation may be reset periodically to avoid the weighting of out-of-date measurements. In another algorithm, a selected number of values are added together to provide an initial average value and thereafter the oldest value is dropped from the average as the newest measured valve is added to it.

As shown in Figs. 8A and 8B, control system 130 (*e.g.*, a computer), in addition to collecting and analyzing data received from chemiluminescence detector 126, controls selection valve 114, valve 122 (or valves 122a and 122b, as shown in Fig. 8C), and scrubber-system valves 125, 125' and 125". Selection valve 114 is controlled by control system 130 to channel samples from multiple sampling ports 112 into the conversion module 6 in a selected order. Valve 122 is controlled to switch between channel A and channel B on the basis of settling times (*i.e.*, the time required for the measured NO value to reach equilibrium following a shift). Preferably, multiple switching cycles are employed for a given sample line and the measurements are averaged, or a running average is employed, to produce a reliable measure of total basic nitrogen compounds, as has been described.

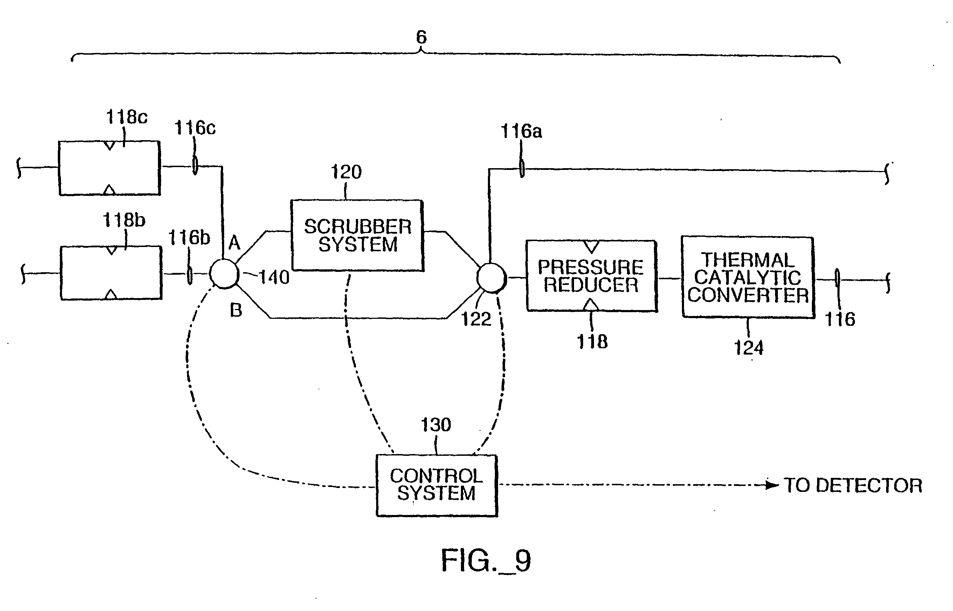

Referring to Fig. 9, in another embodiment, conversion module 6 employs an additional valve 140 at the branching point of channels A and B. Valve 140, in conjunction with valve 122, isolates basic-nitrogen-compound scrubber system 120 and thus prevents the possibility of back-flow diffusion. The amount of sample that is directed by valve 140 to channel A and channel B is controlled by control system 130. In this embodiment, valve 122 is also connected to the vacuum pump by an auxiliary conduit 116a (which, again, is connected, in parallel with sample line 116, to a common vacuum pump) to draw gas through the non-selected channel so that substantially continuous flow conditions are maintained through both channels A and B at all times. Valve 140 is also connected to sample lines 116b and 116c, which are connected, in parallel, upstream from channels A and B. Sample lines 116b and 116c include respective pressure reducers 118b and 118c, which restrict the flow of sample gas to different degrees. When the sample gas is flowing through channel B, valve 140 selects the sample line with the more-restrictive pressure reducer (in this example, sample line 116c) as the source of that gas. The resistance of the more-restrictive pressure reducer is selected to offset the inherent flow restriction generated by the scrubber system 120. While channel B is selected, valve 140 connects sample line 116b, which has the less-restrictive pressure reducer, to supply a flow of gas sample through channel A and on through auxiliary sample line 116a.

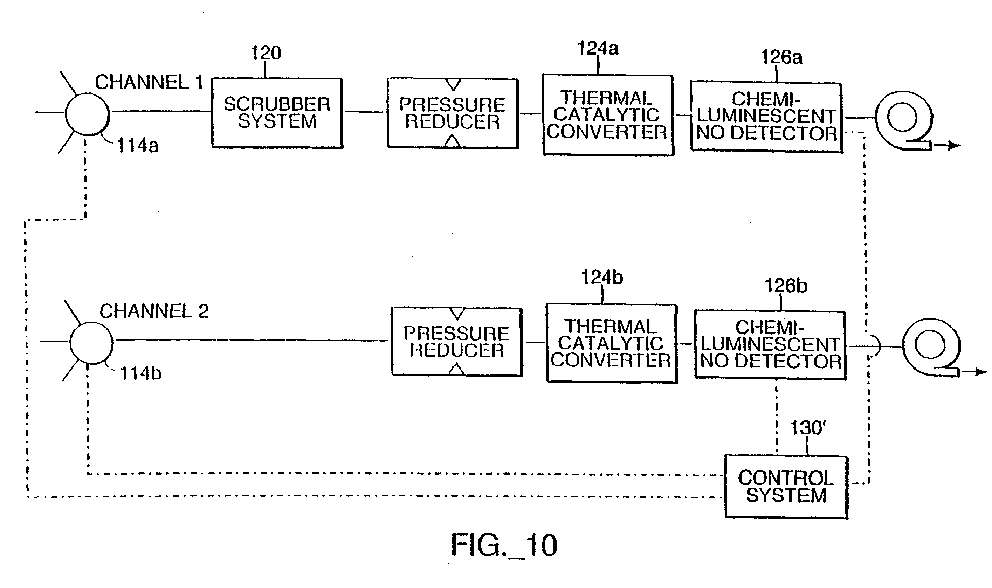

Referring to Fig. 10, in another embodiment, a separate and distinct channel for producing a reference of zero air is operated parallel with another channel for determining the total basic-nitrogen-compound concentration. Sampling point selection valves 114a and 114b, for channels 1 and 2, may be gauged and arranged to simultaneously sample the same location. Channel 1 produces a reference of zero air by directing a sample to basic-nitrogen-compound scrubber system 120 and then to a thermal catalytic converter 124a and chemiluminescence detection device 126a. At the same time, channel 2 directs a sample to a thermal catalytic converter 124b and a chemiluminescence detection device 126b. The NO measurements from channel 1 and 2 are made simultaneously and then compared. This embodiment provides an interference calibration eliminating the effect of fluctuations in ambient NO and $NO_2$ concentrations by determining the actual NO and $NO_2$ concentration at the same time as the total NO response is being measured.

Control system 130' calculates the total basic-nitrogen-compound concentration based on the differences between the two readings from the detectors 126a, 126b. A calibration system (not shown) is employed to compatibly zero the instruments (*e.g.*, to accommodate variations in the converters and detection devices) so that they can operate together. A correction factor based upon, *e.g.*, computer look up of an experience table, can be employed for calibration purposes. A calibration routine can be conducted periodically, and drift trends can be measured and stored to create a dynamic correction algorithm.

This arrangement eliminates the possibility of noise from variations in ambient NO and $NO_2$ concentrations because the instantaneous value of the NO and $NO_2$ concentration is always known and does not change during a calculation cycle. This system, in effect, reduces the time between ambient NO and $NO_2$ measurements to zero, which solves the fundamental problem of fluctuations in NO and $NO_2$ concentrations during a single calculation cycle.

The various embodiments may be implemented in a number of useful ways. The parameters of the basic-nitrogen-compound scrubbers are selected to remove only those basic nitrogen compounds that affect a given process. In one advantageous example, beds of photoresist-coated beads are used as the scrubbers, the photoresist material being selected to correspond to the photoresist material being employed in the process being monitored. Thus, the scrubbers remove those basic nitrogen compounds to which the photoresist process is peculiarly sensitive. In an important case, the scrubbers used in the detection systems of Figs. 8-10 are constructed and arranged to select construction materials for use in a basic-nitrogen-compound-sensitive process (*e.g.*, chemically-amplified photoresist process). In another important case, the detection system of Figs. 8-10 is connected to monitor the performance of a basic-nitrogen-compound gas filter system used to filter gas in a DUV stepper, scanner or coat/develop track. In another important case, the detection system of Figs. 8-10 is connected to monitor the total basic-nitrogen-compound concentration inside a DUV stepper, scanner, or coat/develop track. In another important case, the detection system of Figs. 8-10 is connected to monitor cleanroom concentration of total basic-nitrogen-compound concentration inside a DUV stepper, scanner, or coat/develop track.

In addition to "zero" calibration, discussed above, another calibration important to maintaining accuracy in measurements is a "span" calibration. The term, "span," as used herein, refers to a scaled range of values indicative of the basic-nitrogen-compound concentration in a gas sample. The scale, for example, can range from zero to 100, where "zero" indicates an absence of basic nitrogen compounds and "100" indicates a maximum concentration of basic nitrogen compounds. Over the course of time, detector readings may drift, for example, due to a shift in converter efficiency or a clouding of the optics in the photomultiplier tube.

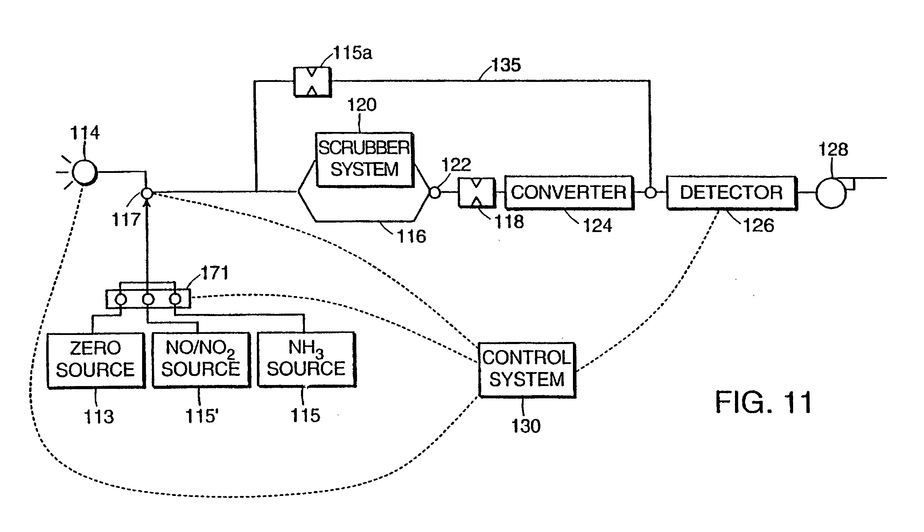

In the embodiment illustrated in Fig. 11, span calibration is performed by a control system 130 which generates commands for directing calibration-gas selection valve 171 and multi-way valves 117 and 122 to circulate zero air from a zero source 113 through the scrubber system 120, converter 124 and detector 126. The reading from the detector 126 is then recorded by the control system 130 to establish a "zero reference." The control system 130 then directs multi-way valves 117 and 122 to circulate a known reference concentration of a basic nitrogen compound (*e.g.*, ammonia) from known-concentration source 115 through the scrubber system 120, converter 124 and detector 126 to generate a "known-concentration reference" from the detector. The control system 130 matches the known-concentration reference with a preset contamination level on a scale of basic-nitrogen-compound concentrations. The preset contamination level can either be at the scale maximum or be at an intermediate number. Regardless, with the known reference concentration of basic nitrogen compound circulating through the conversion module 6 and detector 126, the reading from the detector 126 is again measured by control system 130. The control system 130 then calculates the difference between detector readings as measured for the zero air and as measured for the gas with the known reference concentration of basic nitrogen compound and correlates this difference with the difference in contamination level for the two gas samples. Accordingly, by dividing the difference in detector readings with the difference in contamination levels for the two gas samples, the control system 130 establishes a precise ratio for each unit change of span and the corresponding change in detector reading. When the conversion module 6 is put back into normal operation, the control system 130 then uses this ratio to assign a contamination level within the calibrated span to subsequently-received detector readings. The control system 130 then compares the generated value with a threshold value to determine whether the process is to be shut down due to excessive basic-nitrogen-compound contamination.

Alternatively, the zero source 113 and known-concentration source 115 can be coupled with the conversion module 6 upstream from valve 122 either at valve 114 or at valve 123. If the sources 113 and 115 are coupled with the conversion module 6 at valve 114 or at valve 123, shifts in span due to changes in scrubber efficiency can likewise be factored into the calibration.

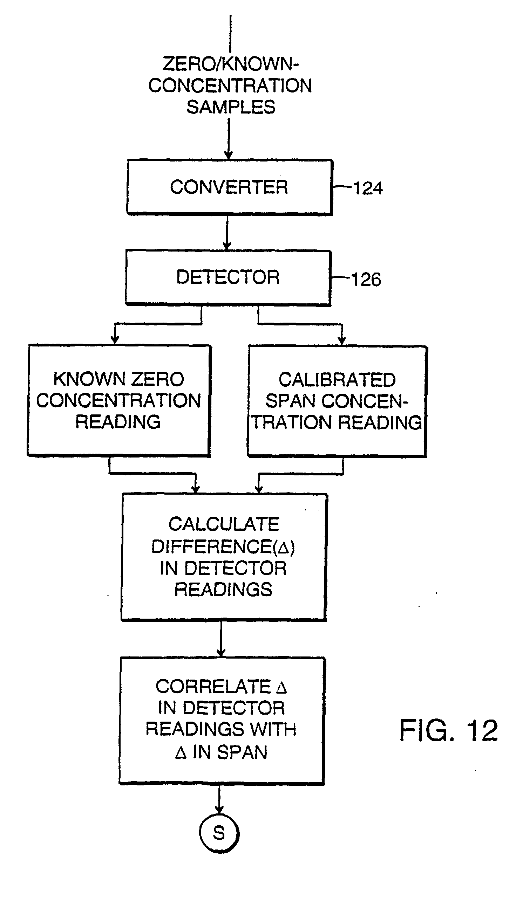

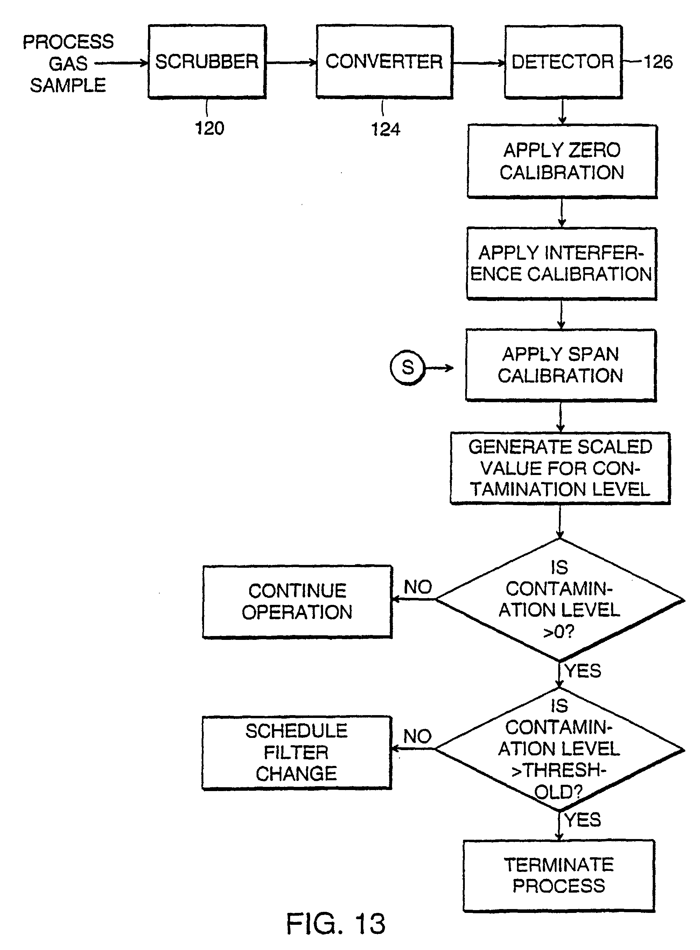

The control system 130 includes a computer having a processor governed by software code stored on a computer-readable medium in communication with the processor, where the software code directs the above-described span calibration process. The steps in this process, as directed by the processor and software code, are further outlined in Figs. 12 and 13. Specifically, the software code directs the processor to generate commands to separately direct gas samples from the zero source and known-concentration source through the convertor and detector. The processor then receives detector readings for each gas sample. Finally, the processor calculates a difference in the detector readings and generates a span calibration value. As further directed by the software code, the processor then applies the span calibration value to subsequent readings for a process gas to generate a scaled value indicative of the basic-nitrogen-compound contamination level for the process gas.

Typically the span calibration of detector readings is performed in coordination with an interference calibration and a zero calibration, discussed herein. Though the frequency of calibrations is situation-specific, the span calibration process typically is performed about once a day. The zero calibration is typically performed more often due to the magnified affect of relative errors at low concentration levels.. although the frequency of the process can be established by monitoring the deviation in span with each calibration and recording each deviation in memory. If little or no deviation in repeated calibrations is observed in reviewing the calibration history, the frequency of performing the calibration of span or any of the other parameters can be reduced.

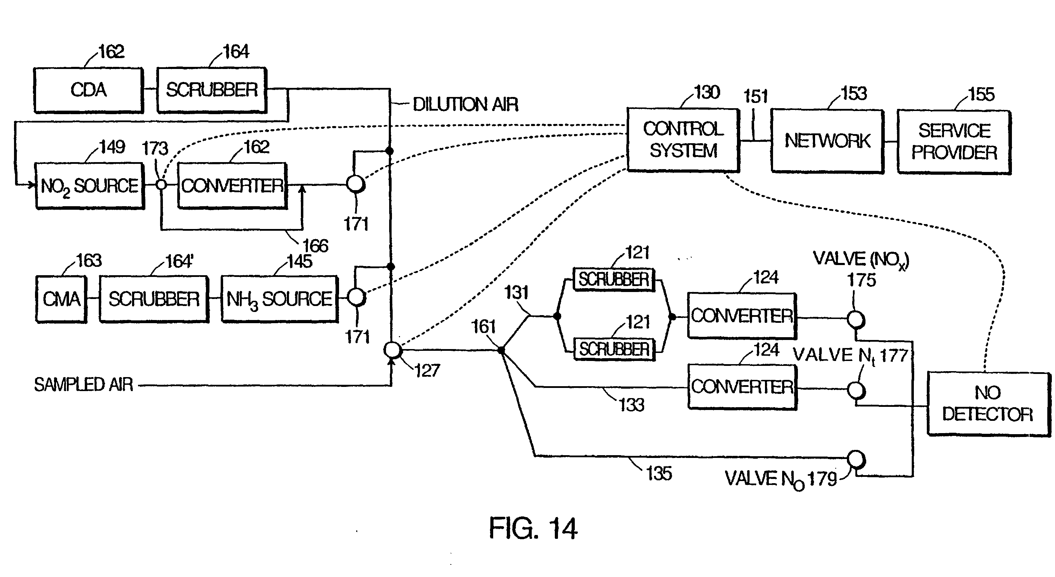

Additional methods of this invention are used for diagnostic evaluation of the functioning of the scrubber, converter and detector subsystems. In the embodiment illustrated in Fig. 14, a plurality of diagnostic gas sources, specifically, a clean dry air source 162, an ammonia gas source 145, an NO gas source 147, and an

$NO_2$ gas source 149 are each coupled with a multi-way valve 127, to which the sampled air 101 is also supplied. Downstream from the multi-way valve 127 is a splitter 161, which directs samples to one of three channels, 131, 133 or 135. The control system 130 directs the system through a four-part sequence useful for both diagnostic evaluation as well as for calibration of detector readings.

The ammonia gas source 145, as well as the NO gas source 147 and $NO_2$ gas source 149, preferably include a liquid precursor, which is vaporized to provide the vapors directed through the system. One or more permeation tubes are provided to allow selective passage of each vaporized precursor into the system. The liquid precursor is contained in a reservoir, and a heater is coupled with a temperature controller that communicates with the control system. Separate reservoirs of liquid $NO_2$ and ammonia liquid are preferably heated to about $30°C$ with a stability of $±0.2°C$. The temperature is tightly controlled due to the exponential increases of vapor pressure with respect to changes in temperature (for example, a $10°C$ increase in either reservoir can result in a doubling of vapor pressure). If the temperature of the reservoir must be shifted outside a pre-established range to provide the necessary vapor to the system, the control system, in accordance with its software, triggers an alarm indicating a system malfunction. For example, the control system can be programmed to trigger an alarm if the temperature of the ammonia reservoir drops below about $25°C$.

The control system and temperature controller cooperate to regulate the heater to heat the liquid precursor to a temperature that will produce a desired vapor pressure. A processor distinct from that used to perform the NO detection algorithm based on detector readings can be used to provide this control over the vapor pressure of the precursor. Accordingly, the processor and processing steps relating directly to the evaluation of NO readings at the detector can be treated as a commodity distinct from the other processes performed in association with this invention and from the other processor(s) associated therewith.

First, the control system 130 directs valves 160, 127, 123 and 139 to direct "zero air" from clean dry air (CDA) source 162 through channel 131 where it passes through a scrubber 121 and converter 124 on its way to NO detector 126. The CDA is derived from ambient air filtered of bases. Before passing through the system, the CDA is preheated to a set temperature and scrubbed by scrubber 164. Meanwhile, the control system 130 monitors readings from the detector 126 to evaluate whether the detector 126 is indicating zero contamination. The phrase, "substantially-zero base contaminants," refers to air with a concentration of base contaminants below the lower detection limit of the system. The lower detection limit is generally about 0.6 parts per billion (ppb).

The control system 130 can be in the form of a personal computer operating software which instructs a processor within the computer to evaluate detector readings and to generate commands based on that evaluation either for providing notice of a system malfunction or for calibrating subsequent detector readings. Depending on the magnitude of the difference from a zero reading provided by the detector 126, the control system 130 can either provide a calibration value to compensate for this difference in subsequent readings or can trigger an alarm, in the case of a significant difference, to indicate a system malfunction. A system malfunction can result from faulty performance of a scrubber 121, converter 124 or detector 126 or from a leak in the system. The alarm can be in the form of a service-call request electronically sent by the control system 130 via line 151 to a network 153 that directs the service-call request to a service provider 155.

Second, the control system 130 directs valves 173, 171, 127 and 179 to direct $NO_2$ from $NO_2$ source 149, where the $NO_2$ is diluted with clean dry air from CDA source 162 to about 100 to about 1,000 ppb $NO_2$ and converted to NO in converter 162 through channel 135 directly to the detector 126. No further conversion is needed because the NO can be measured by the detector 126 without any extra processing. The control system 130 monitors readings from the detector 126 to evaluate whether the detector 126 indicates an NO level matching the known concentration level leaving the converter 162. Again, the control system 130 can either provide a calibration value to compensate for this difference in subsequent readings or can trigger an alarm (*e.g.*, transmitting a service-call request), in the case of a significant difference, to indicate a system malfunction.

As an alternative to the above-described use of an NO source (*e.g.*, an NO canister), NO can be provided by directing $NO_2$ from $NO_2$ source 149 through converter 162, where a molybdenum catalyst catalyzes a reaction converting $NO_2$ to NO, and then through permeation tube 164 which selectively allows NO to pass through.

Third, the control system 130 directs a concentration of about 100 to about 1,000 ppb $NO_2$ from $NO_2$ source 149, with dilution again provided by clean dry air from CDA source 162 through valve 173, branch 166, and then through valves 171, 127, and 175/177, and then through the converter 124 in channels 131 and 133. The control system 130 monitors readings from the detector 126 to measure the difference between the readings and the known $NO_2$ concentration level of the $NO_2$ source 149. As before, the control system 130 can either provide a calibration value to compensate for this difference or can trigger an alarm (*e.g.*, in the form of a transmitted service-call request), in the case of a significant difference, to indicate a system malfunction.

The $NO_2$ source 149 will often include some measure of NO, as well, since the $NO_2$ is typically formed by reacting an excess of NO with $O_3$ in compliance with EPA regulations. The $NO_2$ concentration in the source 149 is measured by subtracting the concentration of NO in the source 149 from the concentration of NO detected at the detector 126.

In channel 131, $NO_2$ flows through at least one scrubber 121 and then on to the converter 124 and detector 126. The scrubbers 121 tend to convert a small percentage of $NO_2$ to NO, which can skew detector readings. Via this procedure, the degree to which that conversion takes place can then be calibrated for in subsequent detector readings.

Fourth, the control system 130 directs valves 171, 127 and 175/177 to direct ammonia from ammonia source 145 in a concentration of about 100 to about 200 parts per billion (ppb) alternately through channels 131 and 133. As an alternative to ammonia, trimethylamine, triethylamine, n-methyl pyrolidnone, cyclohexylamine, diethylaminoethanol, methylamine, dimethylamine, ethanolamine or morpholine can be used. Once again, the control system 130 monitors readings from the detector 126 to evaluate whether the detector 126 is indicating a contamination level matching the concentration level of ammonia leaving the ammonia source 145. The control apparatus can then either provide a calibration value to compensate for this difference or can trigger an alarm (*e.g.*, transmission of a service-call request), in the case of readings evidencing a significant variance from the known ammonia concentration, to indicate a system malfunction. Alternatively, the parallel scrubbers 121 can be replaced with a single scrubber. Generally, the parallel-scrubber system, which is more robust than the single-scrubber system, is preferred when a very weak base is used or with a combination of a weak base and a strong base.

Preferably, the ammonia source 145 supplies "wet" ammonia. "Wet" ammonia includes water vapor, provided by mixing with clean, moist air (CMA) from source 163 directed through scrubber 164', at a concentration approximately matching that of ambient air (*e.g.*, about 40% relative humidity), whereas "dry" ammonia is nearly free of water vapor. The catalyst in converter 124 typically is sensitive to water. Accordingly, sampled air, which typically includes ambient water vapor concentration will, over time, affect the conversion efficiency of the converter 124. Consequently, a converter 124 that has been in use for an extended period will tend to convert ammonia with varying efficiency dependant upon the concentration of water vapor in the gas stream. A failure to appreciate and account for the converter's sensitivity to water vapor concentration can produce calibration errors due to the unaccounted-for drop in conversion efficiency for "wet" samples. By directing "wet" ammonia (with a water-vapor concentration matching that of the sampled gases) through the system, any drop in converter efficiency will be canceled out when comparing measurements. In some embodiments, wet and dry ammonia sources can alternately be directed through the system to monitor this difference.

The software code in one embodiment is developed for the WINDOWS platform (from Microsoft Corp.) using VISUAL C++ (available from Microsoft), which builds a template for the WINDOWS environment into which a library of components for governing and monitoring the methods of this invention are integrated.

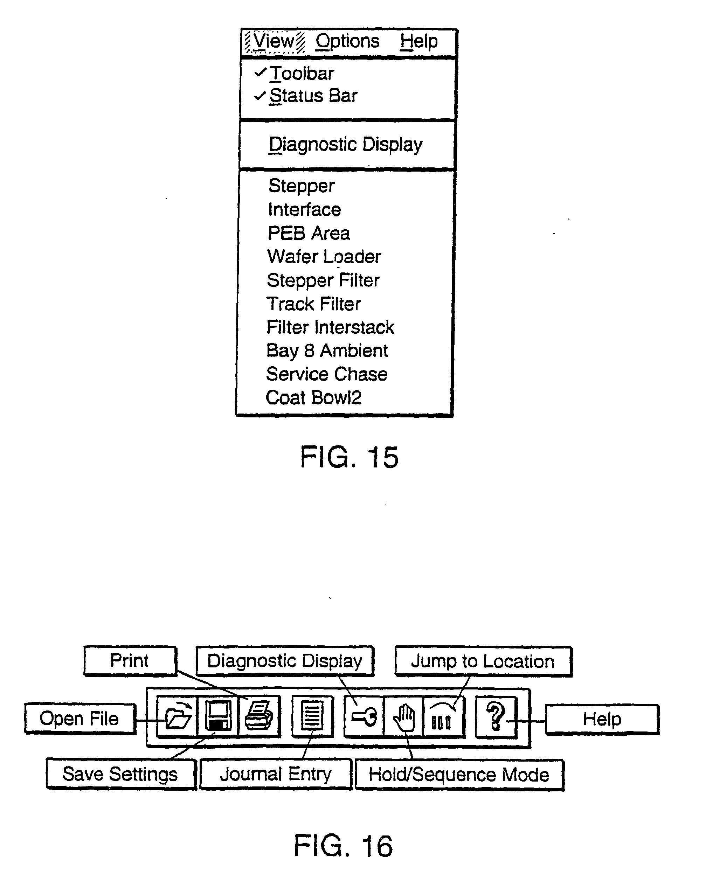

The software allows the operator and administrator to: monitor multiple sample locations (*e.g.*,, locations throughout a semiconductor processing tool); hold and view data received from a single location; jump to a specific location to initiate and view data collection; create data acquisition schedules for several groups; configure sample locations; view real-time data graphically and track historical data on specific location operation. Fig. 15, for example, illustrates a pull-down menu showing a variety of sample locations that can be selected by the operator/administrator. Further, a computer-generated toolbar for selection of various software functions is illustrated in Fig. 16.

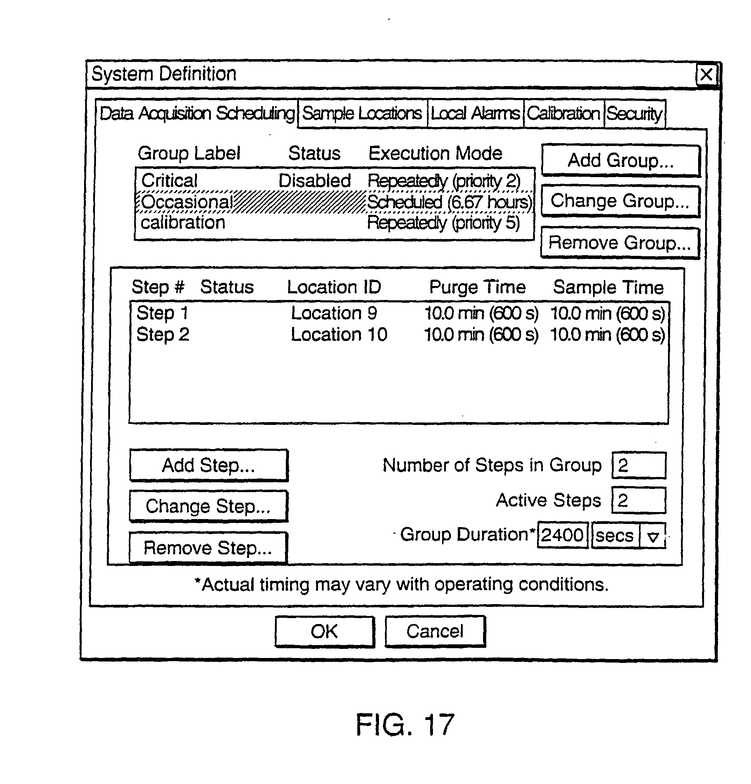

A display is illustrated in Fig. 17, wherein the data acquisition schedule for the system including group label, status indication, execution mode and purge and sample times are displayed.

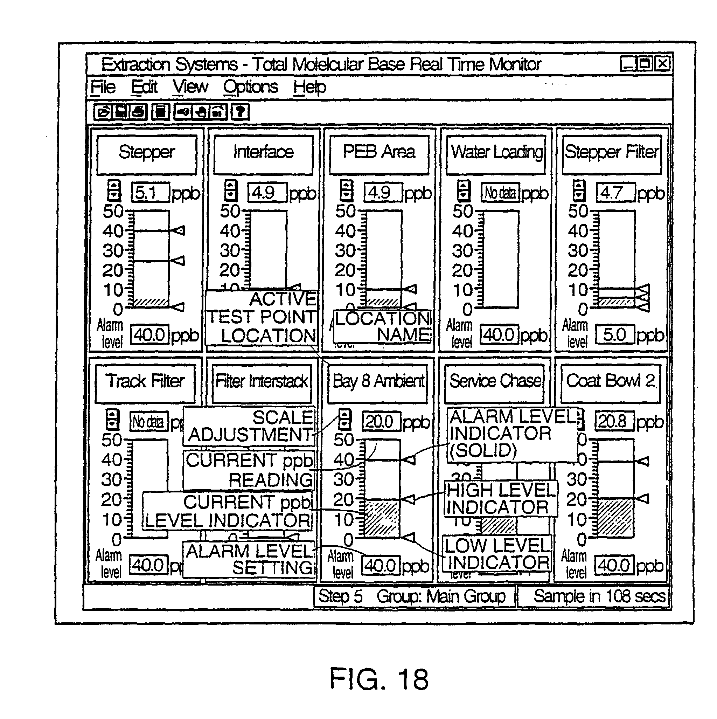

Further, a sample locations display is illustrated in Fig. 18. The display is divided into ten frames, with each frame representing a sample location. The display provides a snapshot view of data as it is captured from all TMB-RTM test point locations in real time, and over a period of time. The computer cycles through all locations and displays the data as it is captured as the solid bar in the center of each frame and as the numerical ppb reading at the top of the frame. As the computer cycles through all sample locations, the active sample location has a white border and background.

The display includes the following features:

1) Location Name: The Sample Location Name is displayed in the box at the top of each frame. Location names are set up in Administrator Mode.

2) Current ppb Reading: The Current ppb Reading is displayed numerically at the top of each location frame.

3) Current ppb Level Indicator: The Current Level Indicator is a graphical representation of the current ppb reading. When the operator double clicks on this indicator, the software launches a real time view of the date, displayed in a graph format.

4) Alarm Level Indicator: The Alarm Level Indicator is represented by a bar with a solid red arrow to the right of the bar. This indicator is a graphical representation of the Alarm Level set by the Administrator. When the Current ppb Level is in an alarm state, the indicator is red.

5) Alarm Level Setting: The Alarm Level Setting is set by the Administrator in Administrator Mode and cannot be adjusted by the user. The setting is displayed in numerical format at the bottom of each location frame.

6) High Level Indicator over time: This indicator displays graphically the high ppb level at each location, over a period of time preset by the Administrator.

7) Low Level Indicator over time: This indicator displays graphically the low ppb level at each location over a period of time preset by the Administrator.

8) Scale Adjustment: There is a scale adjustment available at the top of each frame that allows the user to adjust the scale for that particular frame. This can be helpful if readings are either high or low and the arrows are out of the scale displayed.

Note that when the current ppb level indicator in a frame is read it indicates that there is an alarm condition at that particular sample location.

The software further includes modules and associated interfaces allowing the administrator to create several user groups for monitoring and to define different schedules for acquiring data for each group. Further still, a data acquisition scheduling screen allows the administrator to define the sequence of steps in data acquisition. The following parameters are defined for each step: order in sequence, location, purge time and sample time. "Sample time" is the actual amount of time that a sample is taken at a particular location. "Purge time" is the actual amount of time that the system takes to purge the current sample fo gas from the instrument for the sampled location. Sample and purge times do not have to be the same for each step.

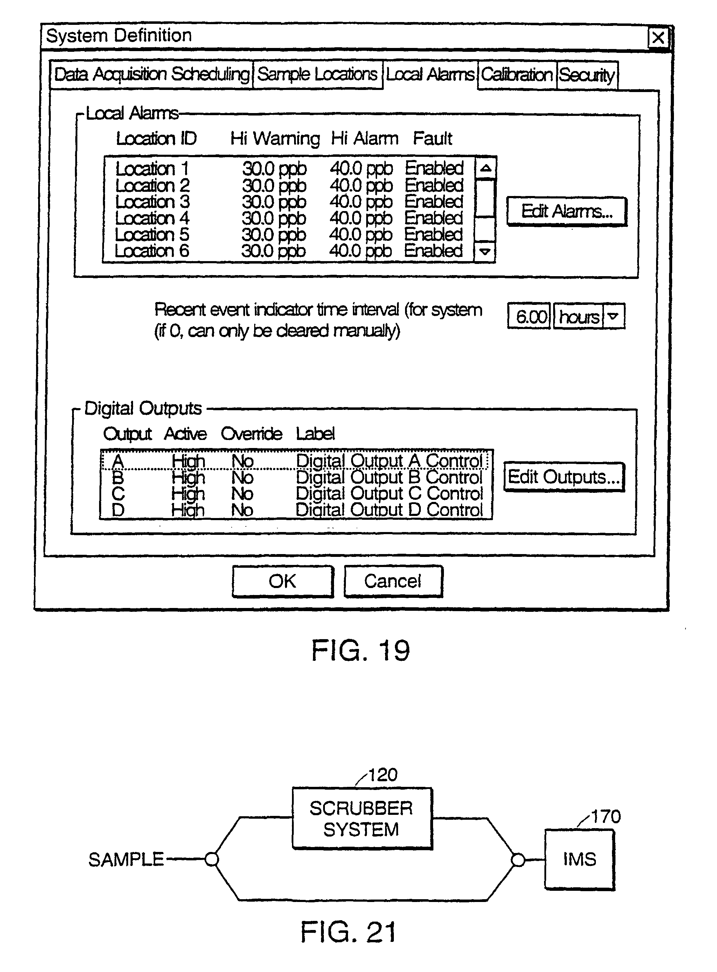

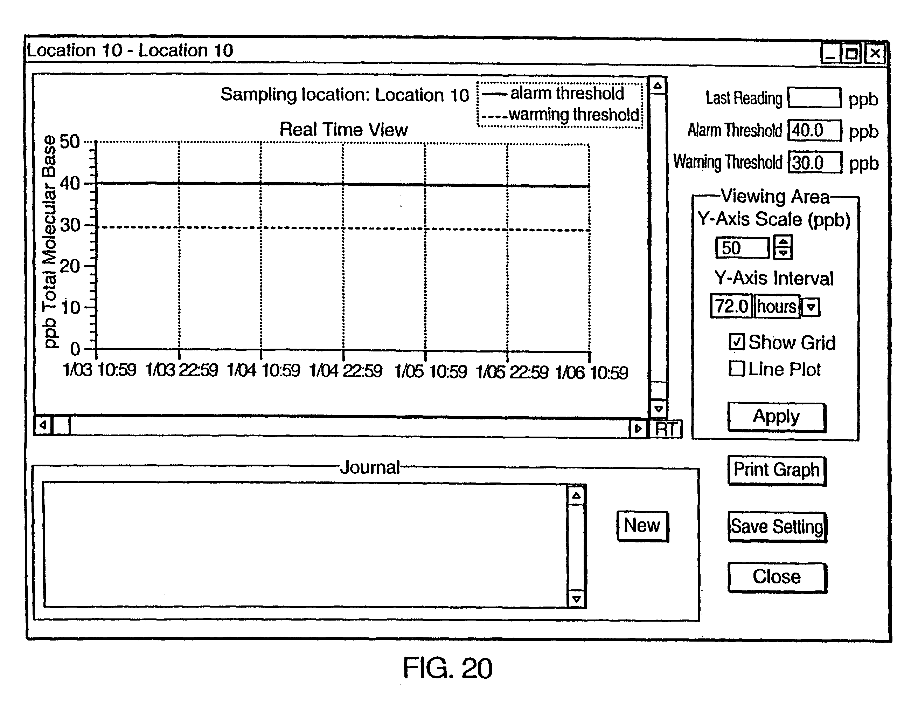

Additional screens are provided in Figs 19 and 20. In Fig. 19, a local alarm screen is illustrated, wherein the high and low alarm functions at each location are defined. Finally, Fig. 20 illustrates a real-time view of a sampling monitor. The horizontal line at 30 pbb represents a warning threshold, and the horizontal line at 40 ppb represents an alarm threshold. In operation, the detected base contaminant level is chart on the graph as a function of time.

In another system of this invention, illustrated in Fig. 21, the detector is an ion mobility spectrometer (IMS) 170 (available from Molecular Analytics, Sparks, MD 21152, USA IMS's, however, are prone to interference; particularly prone are NMP sensors. NMP sensor readings can be skewed by the presence of acetone, for example, *See*, Bernard T. Beauchemin, Jr., *et al*., "Optimization and Utilization of IMS for Deep UV Clean Room Air Monitoring of Amines and NMP," *Proceedings of the Arch Microlithography Symposium, San Diego, Nov. 14-16, 1999*, at 261. Acetone is a common laboratory solvent used for cleaning equipment contaminated with positive photoresist. Because the ion mobility spectra of acetone overlaps with that of NMP, the presence of acetone residue in the system can cause a false positive detection in the IMS or, at higher NMP concentration, trigger a false alarm. The use of a scrubbed/unscrubbed sampling system of this invention, whereby a primary channel is introduced into the IMS and a secondary channel with one or more basic-nitrogen-compound scrubbers 120 (either the single scrubber or parallel scrubber embodiments) is introduced into the IMS, allows for calibration based on this interference. The difference between the two readings provides a measure of the concentration of the basic nitrogen compound in the sampled gas.

## Claims

1. A detection system for detecting basic nitrogen compounds comprising:

> a sensor that detects a concentration of an indicator gas in a gas sample;
> a converter apparatus that converts gaseous nitrogen compounds in the gas sample into the indicator gas, the converter apparatus being coupled with the sensor for delivering the converted gas sample to the sensor;
> a primary channel coupled with the converter apparatus to deliver a target gas sample to the converter apparatus;
> at least one scrubbing channel coupled with the converter apparatus for delivering a reference gas sample to the converter apparatus, the scrubbing channel including a scrubber for removing basic nitrogen compounds from the reference gas sample; and
> a nitrogen-compound gas source coupled with the convertor apparatus and with the sensor, the nitrogen-compound gas source supplying a diagnostic gas including at least one nitrogen compound of a reference concentration through the convertor apparatus and sensor.

2. The detection system of claim 1, and either: a) wherein the nitrogen compound source includes a reservoir supplied with a heater for vaporizing the diagnostic gas, in which case, optionally, further comprising a control system including a processor and computer-executable software code stored on a computer readable medium coupled with the processor for controlling the temperature of the reservoir to generate a desired vapor pressure of the diagnostic gas;
or b) wherein the nitrogen compound is a basic nitrogen compound; in which case, optionally, further comprising a second gas source coupled with the convertor apparatus and with the sensor, the second gas source supplying a second diagnostic gas, the second diagnostic gas being substantially free of basic nitrogen compounds or having a reference concentration of basic nitrogen compounds distinct from that of the diagnostic gas supplied by the basic-nitrogen-compound gas source; in which case, further optionally, either i) wherein the converter apparatus includes a plurality of convertors, and the sensor includes a plurality of detectors, and wherein a first convertor and first detector are coupled to the primary channel, and a second convertor and second detector are coupled to the scrubbing channel,
or ii) further comprising a third channel that directs an unscrubbed, unconverted gas sample to the sensor, in which case, further optionally comprising a third gas source coupled with the sensor, the third gas source supplying a third diagnostic gas including a non-basic nitrogen compound of a reference concentration, in which case optionally wherein the third diagnostic gas supplied by the third gas source includes NO of a reference concentration, in which case, yet further optionally, further comprising a fourth gas source coupled with the sensor, the fourth gas source supplying a fourth diagnostic gas including $NO_2$ of a reference concentration;
or iii) further comprising a control system programmed and configured to:

> generate commands to direct a first gas sample from a first gas source and to direct a second gas sample from a second gas source through the convertor apparatus and sensor;
> receive sensor readings for each gas sample;
> generate a span calibration value based on:

>> the difference in the sensor reading for the first gas sample and the sensor reading for the second gas sample; and
>> the difference in reference concentrations of basic nitrogen compounds in the first and second gas samples; and

> apply the span calibration value to detector readings for a process gas to generate a scaled value indicative of the concentration of basic nitrogen compounds in the process gas, and optionally

further comprising the step of directing the process gas from a photolithography tool to the sensor; in which case, further optionally,
wherein the control system is further programmed to generate an interference calibration value; in which case, further optionally,
wherein the sensor includes at least two detectors and the control system is further programmed and configured to receive substantially-simultaneous detector readings from a pair of detectors and to generate the interference calibration value as a function of the substantially-simultaneous detector readings;
or c) further comprising a control system in communication with the sensor, wherein the control system is programmed to send commands to direct the diagnostic gas through the sensor, and wherein the control system is further programmed to compare detector readings for the diagnostic gas with a known nitrogen-compound concentration in the diagnostic gas, in which case, optionally

wherein the control system is programmed to send commands to alternately direct a first diagnostic gas and a second diagnostic gas through the sensor and to compare detector readings for both 25 diagnostic gases with known nitrogen-compound concentrations in both diagnostic gases.

3. The detection system of claim 2 with option b), wherein the diagnostic gas includes water vapor of an ambient concentration.

4. A span-calibrated method for monitoring basic-nitrogen-compound contamination in sampled gas, comprising the steps of:

> passing a first gas through a converter apparatus including at least one converter that converts gaseous nitrogen compounds in the first gas into an indicator gas, the first gas including at least one gaseous nitrogen compound of a first reference concentration; then
> passing the first gas through a sensor that includes at least one detector that generates a reading of the indicator gas;
> passing a second gas through the converter apparatus, the second gas having a second reference concentration of gaseous nitrogen compounds distinct from the first reference concentration; then
> passing the second gas through the sensor to generate a reading of the indicator gas; and
> generating a span-calibration value based on the difference in detector readings for the first gas and the second gas correlated with the difference in reference concentrations in the first and second gas;
> separating a process gas into a reference gas sample and a target gas sample;
> passing the reference gas sample through a scrubbing channel including at least one scrubber that removes basic nitrogen compounds and then passing the reference gas sample through the converter apparatus and on to the sensor; and
> passing the target gas sample through a primary channel in which the basic-nitrogen-compound contamination level in the target gas sample remains substantially unchanged and then passing the target gas sample through the converter apparatus and on to the sensor;
> using the sensor to generate respective readings of the indicator gas in both the converted reference gas sample and the converted target gas sample; and

comparing the respective detector readings and applying the span calibration value to generate a value indicative of the level of basic-nitrogen-compound contamination in the process gas.

5. The method of claim 4, further comprising the step of passing a third gas sample through a channel free of scrubbers and converter, and optionally, wherein the target gas sample and the reference gas sample are passed through distinct converters in the converter apparatus and through distinct detectors in the sensor, in which case, further optionally

further comprising the step of subtracting the detector reading for the reference gas sample from the detector reading for the target gas sample to calibrate for fluctuations in the concentration of interference gases, in which case, further optionally,

further comprising the step of obtaining the process gas from a photolithography tool, in which case, further optionally,

wherein the detectors simultaneously measure the concentration of indicator gases in the target sample and the reference sample.

6. An interference-calibrated detection system for detecting contaminant gases comprising:

> a primary channel through which a target gas sample is passed, the primary channel having a primary converter for converting gaseous nitrogen compounds in the gas samples into an indicator gas, and a primary detector for detecting the indicator gas; and
> a scrubbing channel through which a reference gas sample is passed, the scrubbing channel including at least one scrubber for removing basic nitrogen compounds from the reference gas sample, a secondary converter for converting gaseous nitrogen compounds in the gas samples into an indicator gas, and a secondary detector for detecting the indicator gas, and optionally,
> further comprising a third channel that directs an unscrubbed, unconverted gas sample to a detector, in which case, optionally,
> further comprising a control system coupled to the detectors and programmed to determine the difference in readings from the primary detector and from the secondary detector to provide an indication of the concentration of basic nitrogen compounds in the target gas sample, in which case, optionally,
>
> wherein the control system is programmed to determine the difference in simultaneously recorded readings from the primary detector and from the secondary detector.

7. An interference-calibrated method for measuring the concentration of basic nitrogen compounds in a target gas sample comprising the steps of:

passing a target gas sample through a primary channel where a primary converter converts gaseous nitrogen compounds in the target gas sample into an indicator gas, and a primary detector generates a reading of the indicator gas; passing a reference gas sample through a scrubbing channel where a scrubber removes basic nitrogen compounds, a secondary converter converts gaseous nitrogen compounds in the reference gas sample into an indicator gas, and a secondary detector generates a reading of the indicator gas; and comparing the readings from the primary detector and secondary detector to provide an indication of basic-nitrogen-compound concentration based on the difference in detector readings.

8. The method of claim 7, wherein simultaneously-recorded readings from the primary detector and secondary detector are compared.

9. A detection system for detecting basic nitrogen compounds comprising:

a converter apparatus that converts a nitrogen compound in a gas sample into an indicator gas; a sensor that detects the indicator gas, the sensor being coupled with the converter apparatus to receive a gas sample from the converter apparatus; a primary channel coupled with the converter apparatus to deliver a target gas sample to the converter apparatus; at least one scrubbing channel including at least one scrubber, the scrubbing channel coupled with the converter apparatus to deliver a reference gas sample to the converter apparatus; and a third channel coupled with the sensor to deliver an unscrubbed, unconverted gas sample to the sensor.

10. The detection system of claim 9 an either a), wherein the sensor includes a plurality of detectors such that all three channels do not share a common detector; or b) wherein the converter apparatus includes at least two converters, with distinct converters positioned in the primary channel and the scrubbing channel;

or c) wherein the scrubbing channel includes a pair of scrubbers joined in parallel.

11. A detection system for detecting a contaminant gas, comprising:

a sensor that detects an indicator gas in a gas sample; a primary channel that delivers a gas sample to the sensor; a plurality of scrubbing channels that delivers a reference gas sample to the detector such that each of the scrubbing channels includes a scrubber that removes a basic nitrogen compound from the reference gas sample; and a converter coupled to the primary channel and to the scrubbing channels that converts a gaseous nitrogen compound in a gas sample into the indicator gas.

12. The detection system of claim 11 and either a), further comprising a purge system coupled to at least one of the scrubbers for purging reversibly-bound basic nitrogen compounds from the scrubber. or b) wherein the scrubbers include a cation exchange medium; or c) wherein the scrubbing channels are connected in parallel; and optionally, further comprising a flow controller positioned for selectively controlling which of the scrubbing channels the sampled gas can flow through to the converter, in which case, optionally, wherein the flow controller is governed by a control system that is programmed to transfer the flow of the reference gas sample reaching the sensor from a scrubbing channel with a contaminated scrubber to a scrubbing channel with a purged scrubber and to then direct a purge gas through the contaminated scrubber, in which case, optionally, wherein the control system is programmed to transfer the flow of the reference gas sample away from one of the scrubbing channels and to purge the scrubber of that scrubbing channel before a weakbase nitrogen compound can penetrate through the scrubber; or, wherein the control system is programmed to alternately transfer the flow of a gas sample between the primary channel, where the gas sample becomes the target gas sample, and one of the scrubbing channels, where the gas sample becomes the reference gas sample; or d) wherein the scrubbers include photoresistcoated beads. or e) further comprising a pressure reducer located between the sensor and the scrubbers. or f) wherein scrubbers are excluded from the primary channel.

**13.** A method for monitoring basic-nitrogen-compound contamination in sampled gas, comprising the steps of:

passing a first reference gas sample through a first scrubber to remove a basic nitrogen compound from the first reference gas sample;

passing a second reference gas sample through a second scrubber to remove a basic nitrogen compound from the second reference gas sample;

purging the first scrubber to remove bound basic nitrogen compounds while passing the second reference gas sample through the second scrubber;

passing the first and second reference gas samples through a converter that converts a gaseous nitrogen compound in the reference gas samples into an indicator gas after the reference gas samples are passed through their respective scrubbers;

passing the first and second reference gas samples through a sensor that detects a concentration of the indicator gas in the reference gas samples after the reference gas samples are passed through the converter;

passing a target gas sample through the converter which converts gaseous nitrogen compounds in the target gas sample into the indicator gas;

passing the target gas sample through the sensor that detects the concentration of the indicator gas in the target gas sample after the target gas sample passes through the converter; and

determining a basic-nitrogen-compound contamination concentration by comparing the detected concentration of the indicator gas in the target gas sample with the detected concentration of the indicator gas in at least one of the reference gas samples.

**14.** The method of claim 13 and either a), wherein a common detection device alternately detects the concentration of the indicator gas in the reference gas sample flowing through the first scrubber and the concentration of the indicator gas in the reference gas sample flowing through the second scrubber;

or b) further comprising the step of alternately purging the first and second scrubbers while maintaining substantially uninterrupted flow of the gas samples to the converter and to the sensor;

or c), wherein the gas samples are taken from a photolithography tool cluster.

**15.** A photolithography system, comprising:

a photolithography tool;

a sensor that detects a concentration of an indicator gas in a gas sample from the photolithography tool;

a primary channel that delivers a target gas sample to the detector;

a plurality of scrubbing channels for delivering a reference gas sample to the detector such that each of the scrubbing channels includes a scrubber that removes a basic nitrogen compound from the reference gas sample;

at least one converter coupled to the primary channel and to the scrubbing channels for converting gaseous nitrogen compounds in the gas samples into the indicator gas; and

a control system that controls the flow of a gas sample among the primary channel and the scrubbing channels.

**16.** The system of claim 14, further comprising a purge system coupled to each of the scrubbers that purges removed basic nitrogen compounds from each of the scrubbers,

wherein the scrubbers include ion exchange media.


**Patentansprüche**

**1.** Nachweissystem zum Nachweisen basischer Stickstoffverbindungen, welches einschließt:

einen Sensor, der eine Konzentration eines Indikatorgases in einer Gasprobe nachweist;

eine Konvertereinrichtung, die gasförmige Stickstoffverbindungen in der Gasprobe in das Indikatorgas umwandelt, wobei die Konvertereinrichtung mit dem Sensor verbunden ist, um dem Sensor die umgewandelte Gasprobe zuzuführen;

einen primären Kanal, der mit der Konvertereinrichtung verbunden ist, um der Konvertereinrichtung eine Zielgasprobe zuzuführen;

wenigstens einen Waschkanal, der mit der Konvertereinrichtung verbunden ist, um der Konvertereinrichtung eine Vergleichsgasprobe zuzuführen, wobei der Waschkanal einen Wäscher zum Entfernen von basischen Stickstoffverbindungen aus der Vergleichsgasprobe beinhaltet; und

eine Gasquelle mit Stickstoffverbindungen, die mit der Konvertereinrichtung und dem Sensor verbunden ist,

wobei die Gasquelle mit Stickstoffverbindungen über die Konvertereinrichtung und den Sensor ein Diagnosegas liefert, das wenigstens eine Stickstoffverbindung mit einer Bezugskonzentration beinhaltet.

**2.** Nachweissystem nach Anspruch 1 und entweder:

a) bei dem die Quelle für Stickstoffverbindungen ein Reservoir beinhaltet, das mit einer Heizeinrichtung zum Verdampfen das Diagnosegases versehen ist, in welchem Fall es wahlfrei

ferner ein Steuersystem einschließt, das einen Prozessor und einen auf einem Computer ausführbaren Softwarecode beinhaltet, der auf einem für einen Computer lesbaren Medium gespeichert ist, das mit dem Prozessor verbunden ist zur Steuerung der Temperatur des Reservoirs, um einen gewünschten Dampfdruck im Diagnosegas zu erzeugen; oder

b) bei dem die Stickstoffverbindung eine basische Stickstoffverbindung ist, in welchem Fall es wahlfrei

ferner eine zweite Gasquelle einschließt, die mit der Konvertereinrichtung und mit dem Sensor verbunden ist, wobei die zweite Gasquelle ein zweites Diagnosegas liefert, wobei das zweite Diagnosegas im wesentlichen frei von basischen Stickstoffverbindungen ist oder eine Bezugskonzentration an basischen Stickstoffverbindungen aufweist, die von der des von der Gasquelle mit basischen Stickstoffverbindungen gelieferten Diagnosegases verschieden ist, in welchem Fall weiter wahlfrei entweder

i) die Konvertereinrichtung eine Mehrzahl von Konvertern beinhaltet und der Sensor eine Mehrzahl von Nachweisgeräten beinhaltet und bei dem ein erster Konverter und ein erstes Nachweisgerät mit dem primären Kanal verbunden sind und ein zweiter Konverter und ein zweites Nachweisgerät mit dem Waschkanal verbunden sind, oder

ii) es ferner einen dritten Kanal einschließt, der eine ungewaschene, nicht umgewandelte Gasprobe zum Sensor leitet, in welchem Fall es weiter wahlfrei

eine dritte Gasquelle einschließt, die mit dem Sensor verbunden ist, wobei die dritte Gasquelle ein drittes Diagnosegas liefert, das eine nicht-basische Stickstoffverbindung mit einer Bezugskonzentration beinhaltet, in welchem Fall wahlfrei das von der dritten Gasquelle gelieferte dritte Diagnosegas NO mit einer Bezugskonzentration beinhaltet, in welchem Fall es noch weiter wahlfrei

ferner eine vierte Gasquelle einschließt, die mit dem Sensor verbunden ist, wobei die vierte Gasquelle ein viertes Diagnosegas liefert, das $NO_2$ mit einer Bezugskonzentration beinhaltet; oder

iii) es ferner ein Steuersystem einschließt, das so programmiert und konfiguriert ist, daß es:

Befehle erzeugt, um eine erste Gasprobe von einer ersten Gasquelle und eine zweite Gasprobe von einer zweiten Gasquelle durch die Konvertereinrichtung und den Sensor zu leiten; Sensorablesungen für jede Gasprobe empfängt; einen Kalibrierungswert für den Meßbereich erzeugt, basierend auf:

der Differenz der Sensorablesung für die erste Gasprobe und der Sensorablesung für die zweite Gasprobe; und der Differenz in den Bezugskonzentrationen der basischen Stickstoffverbindungen in der ersten und zweiten Gasprobe; und

den Kalibrierungswert für den Meßbereich auf Ablesungen des Nachweisgerätes von einem Prozeßgas anwendet, um einen maßstabgetreuen Wert zu erzeugen, der die Konzentration basischer Stickstoffverbindungen im Prozeßgas angibt, und das wahlfrei ferner den Schritt des Leitens des Prozeßgases von einem Photolithographiewerkzeug zum Sensor einschließt; in welchem Fall weiter wahlfrei das Steuersystem ferner so programmiert ist, daß es einen Störungskalibrierungswert erzeugt, in welchem Fall weiter wahlfrei

der Sensor wenigstens zwei Nachweisgeräte umfaßt und das Steuersystem ferner so programmiert und konfiguriert ist, daß es im wesentlichen gleichzeitige Nachweisgeräteablesungen von einem Paar von Nachweisgeräten empfängt und den Störungskalibrierungswert abhängig von den im wesentlichen gleichzeitigen Nachweisgeräteablesungen erzeugt; oder

c) das ferner ein Steuersystem in Verbindung mit dem Sensor einschließt,

bei dem das Steuersystem so programmiert ist, daß es Befehle sendet, um das Diagnosegas durch den Sensor zu leiten, und wobei das Steuersystem weiterhin so programmiert ist, um Nachweisgeräteablesungen für das Diagnosegas mit einer bekannten Konzentration von Stickstoffverbindungen in dem Diagnosegas zu vergleichen, in welchem Fall wahlfrei das Steuersystem so programmiert ist, daß es Befehle sendet, um abwechselnd ein erstes Diagnosegas und ein zweites Diagnosegas durch den Sensor zu leiten, und Nachweisgeräteablesungen für beide Diagnosegase mit bekannten Konzentrationen von Stickstoffverbindungen in beiden Diagnosegasen vergleicht.

3. Nachweissystem nach Anspruch 2 mit Option b), bei dem das Diagnosegas Wasserdampf mit einer Umgebungskonzentration beinhaltet.

4. Verfahren mit Meßbereichskalibrierung zum Überwachen einer aus basischen Stickstoffverbindungen bestehenden Verunreinigung in Gasproben, das die Schritte einschließt:

Führen eines ersten Gases durch eine Konvertereinrichtung, die wenigstens einen Konverter beinhaltet, der gasförmige Stickstoffverbindungen im ersten Gas in ein Indikatorgas umwandelt, wobei das erste Gas wenigstens eine gasförmige Stickstoffverbindung mit einer ersten Bezugskonzentration beinhaltet; dann

Führen des ersten Gases durch einen Sensor, der wenigstens ein Nachweisgerät beinhaltet, das eine Ablesung für das Indikatorgas erzeugt;

Führen eines zweiten Gases durch die Konvertereinrichtung, wobei das zweite Gas eine zweite Bezugskonzentration an gasförmigen Stickstoffverbindungen aufweist, die sich von der ersten Bezugskonzentration unterscheidet; und sodann

Führen des zweiten Gases durch den Sensor, um eine Ablesung für das Indikatorgas zu erzeugen; und

Erzeugen eines Kalibrierungswertes für den Meßbereich basierend auf der Differenz der Nachweisgeräteablesungen für das erste Gas und das zweite Gas, die mit der Differenz in den Bezugskonzentrationen im ersten und zweiten Gas korreliert ist;

Trennen eines Prozeßgases in eine Vergleichsgasprobe und eine Zielgasprobe;

Führen der Vergleichsgasprobe durch einen Waschkanal, der wenigstens einen Wäscher beinhaltet, der basische Stickstoffverbindungen entfernt, und dann Führen der Vergleichsgasprobe durch die Konvertereinrichtung und weiter zum Sensor; und

Führen der Zielgasprobe durch einen primären Kanal, in dem der Grad an Verunreinigung mit basischen Stickstoffverbindungen in der Zielgasprobe im wesentlichen unverändert bleibt, und dann Führen der Zielgasprobe durch die Konvertereinrichtung und weiter zum Sensor;

Verwenden des Sensors, um jeweilige Ablesungen für das Indikatorgas in sowohl der umgewandelten Vergleichsgasprobe als auch der umgewandelten Zielgasprobe zu erzeugen; und

Vergleichen der jeweiligen Nachweisgeräteablesungen und Anwenden des Kalibrierungswertes für den Meßbereich, um einen Wert zu erzeugen, der den Grad an Verunreinigung mit basischen Stickstoffverbindungen im Prozeßgas angibt.

5. Verfahren nach Anspruch 4, das ferner den Schritt des Führens einer dritten Gasprobe durch einen von Wäschern und Konvertern freien Kanal einschließt; und wahlfrei

bei dem die Zielgasprobe und die Vergleichsgasprobe durch getrennte Konverter in der Konvertereinrichtung und durch getrennte Nachweisgeräte im Sensor geführt werden, in welchem Fall es wahlfrei

ferner den Schritt des Subtrahierens der Nachweisgeräteablesung für die Vergleichsgasprobe von der Nachweisgeräteablesung für die Zielgasprobe einschließt, um für Schwankungen der Konzentrationen von Störungsgasen zu kalibrieren, in welchem Fall es weiter wahlfrei

ferner den Schritt des Erlangens des Prozeßgases von einem Photolithographiewerkzeug einschließt, in welchem Fall weiter wahlfrei

die Nachweisgeräte gleichzeitig die Konzentration an Indikatorgasen in der Zielprobe und der Vergleichsprobe messen.

6. Störungskalibriertes Nachweissystem zum Nachweisen von Verunreinigungsgasen, welches einschließt:

einen primären Kanal, durch den eine Zielgasprobe geführt wird, wobei der primäre Kanal einen primären Konverter zum Umwandeln von gasförmigen Stickstoffverbindungen in den Gasproben in ein Indikatorgas und ein primäres Nachweisgerät zum Nachweisen des Indikatorgases aufweist; und

einen Waschkanal, durch den eine Vergleichsgasprobe geführt wird, wobei der Waschkanal wenigstens einen Wäscher zum Entfernen basischer Stickstoffverbindungen aus der Vergleichsgasprobe, einen sekundären Konverter zum Umwandeln von gasförmigen Stickstoffverbindungen in den Gasproben in ein Indikatorgas und ein sekundäres Nachweisgerät zum Nachweisen des Indikatorgases beinhaltet, und wahlfrei

das ferner einen dritten Kanal einschließt, der eine ungewaschene, nicht umgewandelte Gasprobe zu einem Nachweisgerät leitet, in welchem Fall es wahlfrei

ferner ein Steuersystem einschließt, das mit den Nachweisgeräten verbunden ist und so programmiert ist, daß es die Differenz der Ablesungen vom primären Nachweisgerät und vom sekundären Nachweisgerät ermittelt, um eine Angabe für die Konzentration an basischen Stickstoffverbindungen in der Zielgasprobe bereitzustellen, in welchem Fall wahlfrei das Steuersystem so programmiert ist, daß es die Differenz der gleichzeitig aufgezeichneten Ablesungen vom primären Nachweisgerät und vom sekundären Nachweisgerät ermittelt.

7. Störungskalibriertes Verfahren zum Messen der Konzentration basischer Stickstoffverbindungen in einer Zielgasprobe, das die Schritte einschließt:

Führen einer Zielgasprobe durch einen primären Kanal, wo ein primärer Konverter gasförmige Stickstoffverbindungen in der Zielgasprobe in ein Indikatorsgas umwandelt und ein primäres Nachweisgerät eine Ablesung für das Indikatorgas erzeugt;

Führen einer Vergleichsgasprobe durch einen Waschkanal, wo ein Wäscher basische Stickstoffverbindungen entfernt, ein sekundärer Konverter gasförmige Stickstoffverbindungen in der Vergleichsgasprobe in ein Indikatorgas umwandelt und ein sekundäres Nachweisgerät eine Ablesung des Indikatorgases erzeugt; und

Vergleichen der Ablesungen vom primären Nachweisgerät und sekundären Nachweisgerät, um eine Angabe der Konzentration an basischen Stickstoffverbindungen basierend auf der Differenz der Nachweisgeräteablesungen bereitzustellen.

8. Verfahren nach Anspruch 7, bei dem gleichzeitig aufgezeichnete Ablesungen vom primären Nachweisgerät und sekundären Nachweisgerät verglichen werden.

9. Nachweissystem zum Nachweisen basischer Stickstoffverbindungen, welches einschließt:

eine Konvertereinrichtung, die eine Stickstoffverbindung in einer Gasprobe in ein Indikatorgas umwandelt;

einen Sensor, der das Indikatorgas nachweist, wobei der Sensor mit der Konvertereinrichtung verbunden ist, um die Gasprobe von der Konvertereinrichtung zu erhalten;

einen primären Kanal, der mit der Konvertereinrichtung verbunden ist, um der Konvertereinrichtung eine Zielgasprobe zuzuführen;

wenigstens einen Waschkanal, der wenigstens einen Wäscher beinhaltet, wobei der Waschkanal mit der Konvertereinrichtung verbunden ist, um der Konvertereinrichtung eine Vergleichsgasprobe zuzuführen; und

einen dritten Kanal, der mit dem Sensor verbunden ist, um dem Sensor eine ungewaschene, nicht umgewandelte Gasprobe zuzuführen.

10. Nachweissystem nach Anspruch 9 und entweder wobei

a) der Sensor eine Mehrzahl von Nachweisgeräten beinhaltet, so daß sich alle drei Kanäle nicht ein gemeinsames Nachweisgerät teilen;
b) die Konvertereinrichtung wenigstens zwei Konverter beinhaltet, wobei getrennte Konverter im primären Kanal und im Waschkanal angeordnet sind; oder
c) der Waschkanal ein Paar Wäscher beinhaltet, die parallel geschaltet sind.

11. Nachweissystem zum Nachweisen eines Verunreinigungsgases, welches einschließt:

einen Sensor, der ein Indikatorgas in einer Gasprobe nachweist;

einen primären Kanal, der dem Sensor eine Gasprobe zuführt;

eine Mehrzahl von Waschkanälen, die dem Nachweisgerät eine Vergleichsgasprobe zuführt, so daß jeder der Waschkanäle einen Wäscher beinhaltet, der eine basische Stickstoffverbindung aus der Vergleichsgasprobe entfernt; und

einen mit dem primären Kanal und den Waschkanälen verbundenen Konverter, der eine gasförmige Stickstoffverbindung in einer Gasprobe in ein Indikatorgas umwandelt.

12. Nachweissystem nach Anspruch 11 und entweder

a) das ferner ein Spülsystem einschließt, das mit wenigstens einem der Wäscher verbunden ist, um reversibel gebundene basische Stickstoffverbindungen aus dem Wäscher zu spülen; oder
b) bei dem die Wäscher ein Kationenaustauschmittel beinhaltet; oder
c) bei dem die Waschkanäle parallel geschaltet sind; und das wahlfrei

ferner einen Durchflußregler einschließt, das dafür angeordnet ist, selektiv zu steuern, durch welchen der Waschkanäle das geprüfte Gas zum Konverter strömen kann, in welchem Fall wahlfrei der Durchflußregler von einem Steuersystem gesteuert wird, das so programmiert ist, daß es den Strom der Vergleichsgasprobe, der den Sensor aus einem Waschkanal mit einem verunreinigten Wäscher erreicht, zu einem Wachkanal mit einem gespülten Wäscher überführt und dann ein Spülgas durch den verunreinigten Wäscher leitet, in welchem Fall wahlfrei das Steuersystem so programmiert ist, daß es den Strom der Vergleichsgasprobe von einem der Waschkanäle weg führt und den Wäscher dieses Waschkanals spült, bevor eine schwach basische Stickstoffverbindung durch den Wäscher eindringen kann; oder bei dem das Steuersystem so programmiert ist, daß es abwechselnd den Strom einer Gasprobe zwischen dem primären Kanal, wo die Gasprobe die Zielgasprobe wird, und einem der Waschkanäle überführt, wo die Gasprobe die Vergleichsgasprobe wird; oder

d) bei dem die Wäscher mit Photoresist beschichtete Kugeln beinhalten; oder

e) das ferner einen Druckminderer einschließt, der sich zwischen dem Sensor und den Wäschern befindet; oder

f) bei dem Wäscher vom primären Kanal ausgeschlossen sind.

13. Verfahren zum Überwachen einer Verunreinigung aus basischen Stickstoffverbindungen in Gasproben, das die Schritte einschließt:

Führen einer ersten Vergleichsgasprobe durch einen ersten Wäscher, um eine basische Stickstoffverbindung aus der ersten Vergleichsgasprobe zu entfernen;
Führen einer zweiten Vergleichsgasprobe durch einen zweiten Wäscher, um eine basische Stickstoffverbindung aus der zweiten Vergleichsgasprobe zu entfernen;
Spülen des ersten Wäschers, um gebundene basische Stickstoffverbindungen zu entfernen, während die zweite Vergleichsgasprobe durch den zweiten Wäscher geführt wird;
Führen der ersten und zweiten Vergleichsgasproben durch einen Konverter, der eine gasförmige Stickstoffverbindung in den Vergleichsgasproben in ein Indikatorgas umwandelt, nachdem die Vergleichsgasproben durch ihre jeweiligen Wäscher geführt wurden;
Führen der ersten und zweiten Vergleichsgasproben durch einen Sensor, der eine Konzentration des Indikatorgases in den Vergleichsgasproben nachweist, nachdem die Vergleichsgasproben durch den Konverter geführt wurden;
Führen einer Zielgasprobe durch den Konverter, der gasförmige Stickstoffverbindungen in der Zielgasprobe in das Indikatorgas umwandelt;
Führen der Zielgasprobe durch den Sensor, der die Konzentration des Indikatorgases in der Zielgasprobe nachweist, nachdem die Zielgasprobe durch den Konverter hindurchgeht; und
Nachweisen einer Konzentration der Verunreinigung mit basischen Stickstoffverbindungen, indem die nachgewiesene Konzentration des Indikatorgases in der Zielgasprobe mit der nachgewiesenen Konzentration des Indikatorgases in wenigstens einer der Vergleichsgasproben verglichen wird.

14. Verfahren nach Anspruch 13 und entweder

a) bei dem eine gemeinsame Nachweiseinrichtung abwechselnd die Konzentration des Indikatorgases in der Vergleichsgasprobe, die durch den ersten Wäscher strömt, und die Konzentration des Indikatorgases in der Vergleichsgasprobe, die durch den zweiten Wäscher strömt, nachweist; oder
b) das ferner den Schritt des abwechselnden Spülens des ersten und zweiten Wäschers einschließt, während ein im wesentlichen ununterbrochener Strom der Gasproben zum Konverter und zum Sensor aufrechterhalten wird; oder
c) bei dem die Gasproben von einer Ansammlung von Photolithographiewerkzeugen genommen werden.

15. Photolithographiesystem, welches einschließt:

ein Photolithographiewerkzeug;
einen Sensor, der eine Konzentration eines Indikatorgases in einer Gasprobe aus dem Photolithographiewerkzeug nachweist;
einen primären Kanal, der dem Nachweisgerät eine Zielgasprobe zuführt;
eine Mehrzahl von Waschkanälen, um dem Nachweisgerät eine Vergleichsgasprobe zuzuführen, so daß jeder der Waschkanäle einen Wäscher beinhaltet, der eine basische Stickstoffverbindung aus der Vergleichsgasprobe entfernt;
wenigstens einen Konverter, der mit dem primären Kanal und den Waschkanälen verbunden ist, um gasförmige Stickstoffverbindungen in den Gasproben in das Indikatorgas umzuwandeln; und
ein Steuersystem, das den Strom einer Gasprobe zwischen dem primären Kanal und den Waschkanälen steuert.

16. System nach Anspruch 14, das ferner ein mit jedem der Wäscher verbundenes Spülsystem einschließt, das entfernte basische Stickstoffverbindungen aus jedem der Wäscher herausspült, bei dem die Wäscher Ionenaustauschmittel beinhalten.

**Revendications**

1. Système de détection servant à détecter des composés d'azote basiques comprenant :

    un capteur qui détecte une concentration d'un gaz indicateur dans un échantillon de gaz,
    un dispositif à convertisseurs qui convertit des composés d'azote gazeux contenus dans l'échantillon de gaz dans le gaz indicateur, le dispositif à convertisseurs étant couplé au capteur pour délivrer l'échantillon de gaz converti au capteur;
    un canal primaire couplé au dispositif à convertisseurs pour délivrer un échantillon de gaz cible au dispositif à convertisseurs;
    au moins un canal d'épuration couplé au dispositif à convertisseurs pour délivrer un échantillon de gaz de référence au dispositif à convertisseurs, le canal d'épuration comprenant un épurateur servant à retirer les composants d'azote basiques de l'échantillon de gaz de référence; et
    une source de gaz formée de composés d'azote couplée au dispositif à convertisseurs et au capteur, la source de gaz de composés d'azote délivrant un gaz de diagnostic incluant au moins un composé d'azote présent en une concentration de référence par l'intermédiaire du dispositif à convertisseurs et du capteur.

2. Système de détection selon la revendication 1, et :

    a) dans lequel la source de composés d'azote inclut un réservoir alimenté par un dispositif de chauffage servant à vaporiser un gaz de diagnostic, auquel cas, facultativement,
        ledit système de commande comporte en outre un processeur et un code logiciel pouvant être exécuté par ordinateur et mémorisé sur un support lisible par ordinateur et couplé au processeur pour commander la température du réservoir de manière à produire une pression de vapeur désirée du gaz de diagnostic; ou
    b) dans lequel le composé d'azote est un composé d'azote basique; auquel cas, facultativement,
        le système de détection comporte en outre une seconde source de gaz couplé au dispositif à convertisseurs et au capteur, la seconde source de gaz délivrant un second gaz de diagnostic, le second gaz de diagnostic étant essentiellement exempt de composés d'azote basiques ou possédant une concentration de référence de composés d'azote basiques distincte de celle du gaz de diagnostic envoyé par la source de gaz formé de composés d'azote basiques; auquel cas, en outre facultativement,

    i) le dispositif à convertisseurs inclut une pluralité de convertisseurs et le capteur inclut une pluralité de détecteurs, et le premier convertisseur et le premier détecteur sont couplés au canal primaire, et un convertisseur et un second détecteur sont couplés au canal d'épuration, ou
    ii) le système de détection comporte en outre un troisième canal qui dirige un échantillon de gaz non épuré et non converti en direction du capteur, auquel cas, en outre facultativement,

    le système de détection comporte une troisième source de gaz couplée au capteur, la troisième source de gaz délivrant un troisième gaz de diagnostic incluant un composé d'azote non basique présentant une concentration de référence, auquel cas, facultativement,
    le troisième gaz de diagnostic envoyé par la troisième source de gaz inclut du NO présent en une concentration de référence, auquel cas, en outre facultativement,
    le système de détection comprend en outre une quatrième source de gaz couplée au capteur, la quatrième source de gaz délivrant un quatrième gaz de diagnostic incluant du $NO_2$ présent en une concentration de référence; ou

    iii) le système de détection comporte en outre un système de commande programmé et configuré de manière à :

        générer des commandes pour diriger un premier échantillon de gaz depuis une première source de gaz et diriger un second échantillon de gaz depuis une seconde source de gaz à travers le dispositif à convertisseurs et le capteur;
        recevoir des indications du capteur pour chaque échantillon de gaz;
        générer une valeur d'étalonnage de portée sur la base :

            de la différence entre les indications du capteur pour le premier échantillon de gaz et les indications du capteur pour le second échantillon de gaz; et
            de la différence des concentrations de référence des composés d'azote basiques dans les premier et second échantillons de gaz; et

        appliquer la valeur d'étalonnage de portée aux indications des capteurs pour un gaz de traitement pour la production d'une valeur étalonnée indicative de la concentration de composés d'azote basiques dans le gaz de traitement, et facultativement,

le système de détection comporte en outre l'étape consistant à diriger le gaz de traitement depuis un outil de photolithographie en direction du capteur; auquel cas en outre facultativement,

le système de commande est en outre programmé de manière à produire une valeur d'étalonnage d'interférence; auquel cas, en outre facultativement, le capteur inclut au moins deux détecteurs et le système de commande est en outre programmé et configuré de manière à recevoir des indications essentiellement simultanées de la part d'une paire de détecteurs et pour générer la valeur d'étalonnage d'interférence en fonction des indications essentiellement simultanées des détecteurs; ou

c) le système de détection comporte en outre un système de commande en communication avec le capteur, dans lequel le système de commande est programmé pour envoyer des commandes pour diriger le gaz de diagnostic à travers le capteur, et dans lequel le système de commande est en outre programmé pour comparer des indications du détecteur où le gaz de diagnostic a une concentration connue de composés d'azote dans le gaz de diagnostic, auquel cas, facultativement,

le système de commande est programmé pour envoyer des commandes de manière à diriger alternativement un premier gaz ainsi qu'un second gaz de diagnostic à travers le capteur et comparer les indications des détecteurs pour les deux gaz de diagnostic, avec des concentrations connues de composés d'azote dans les deux gaz de diagnostic.

3. Système de détection selon la revendication 2 avec l'option b), dans lequel le gaz de diagnostic inclut de la vapeur d'eau avec une concentration ambiante.

4. Procédé à portée étalonnée pour contrôler une contamination d'un gaz échantillonné par des composés d'azote basiques, comprenant les étapes consistant à :

faire passer un premier gaz dans un dispositif à convertisseurs incluant au moins un convertisseur qui convertit des composés d'azote gazeux contenus dans le premier gaz en un gaz indicateur, le premier gaz incluant au moins un composé d'azote gazeux présent avec une première concentration de référence; puis

faire passer le premier gaz à travers un capteur qui inclut au moins un détecteur qui génère une indication du gaz indicateur,

faire passer un second gaz dans le dispositif à convertisseurs, le second gaz possédant une seconde concentration de référence de composés d'azote gazeux, distincte de la première concentration de référence, puis

faire passer le second gaz à travers le capteur pour générer une indication du gaz indicateur; et

générer une valeur d'étalonnage de portée sur la base de la différence des indications des détecteurs pour le premier gaz et le second gaz, corrélée à la différence des concentrations de référence dans les premier et second gaz;

séparer un gaz de traitement en un échantillon de gaz de référence et en un échantillon de gaz cible;

faire passer l'échantillon de gaz de référence dans un canal d'épuration incluant au moins un épurateur qui élimine des composés d'azote basiques, puis faire passer l'échantillon de gaz de référence dans le dispositif à convertisseurs et sur le capteur; et

faire passer l'échantillon de gaz cible dans un canal primaire, dans lequel le niveau de contamination par les composés d'azote basique dans l'échantillon de gaz cible reste essentiellement inchangé, puis faire passer l'échantillon de gaz cible dans le dispositif à convertisseurs et sur le capteur;

utiliser le capteur pour produire des indications respectives du gaz indicateur à la fois dans l'échantillon de gaz de référence converti et dans l'échantillon de gaz cible converti; et

comparer les indications respectives des détecteurs et appliquer la valeur d'étalonnage de portée pour produire une valeur indicative du niveau de la contamination par des composés d'azote basiques dans le gaz de traitement.

5. Procédé selon la revendication 4, comprenant en outre l'étape consistant à faire passer un troisième échantillon de gaz dans un canal exempt de dispositifs d'épuration et de convertisseurs, et facultativement,

dans laquelle l'échantillon de gaz cible et l'échantillon de gaz de référence traversent des convertisseurs distincts dans le dispositif à convertisseurs et dans des détecteurs distincts dans le capteur, auquel cas, en outre facultativement,

le procédé comprend en outre l'étape consistant à soustraire l'indication du détecteur pour l'échantillon de gaz de référence de l'indication du détecteur pour l'échantillon de gaz cible pour réaliser un étalonnage pour des fluctuations de la concentration de gaz d'interférence, auquel cas, en outre facultativement,

le procédé comprend en outre l'étape consistant à obtenir le gaz de traitement à partir d'un outil de photolithographie, auquel cas, en outre facultativement,

les détecteurs mesurent simultanément la concentration de gaz indicateurs dans l'échantillon cible et dans l'échantillon de référence.

6. Système de détection étalonné du point de vue interférence pour la détection de gaz polluants, comprenant :

un canal primaire, dans lequel on fait passer un échantillon de gaz cible, le canal primaire possédant un convertisseur primaire pour convertir des composés d'azote gazeux contenus dans les échantillons de gaz en un gaz indicateur, et un détecteur primaire pour détecter le gaz indicateur; et
un canal d'épuration, dans lequel on fait passer un échantillon de gaz de référence, le canal d'épuration incluant au moins un épurateur pour retirer des composés d'azote basiques de l'échantillon de gaz de référence, un convertisseur secondaire pour convertir des composés d'azote gazeux contenus dans les échantillons de gaz dans un gaz indicateur, et un détecteur secondaire pour détecter les gaz indicateurs, et facultativement,
comprenant en outre un troisième canal qui dirige un échantillon de gaz non épuré et non converti à un détecteur, auquel cas, facultativement,
le système comporte en outre un système de commande couplé aux détecteurs et programmé pour déterminer la différence des indications fournies par le détecteur primaire et par le détecteur secondaire de manière à obtenir une indication de la concentration des composés d'azote basiques dans l'échantillon de gaz cible, auquel cas, facultativement,
le système de commande est programmé pour déterminer la différence entre les indications enregistrées simultanément à partir du détecteur primaire et à partir du détecteur secondaire.

7. Procédé étalonné du point de vue interférence pour mesurer la concentration de composés d'azote basiques dans un échantillon de gaz cible, comprenant les étapes consistant à :

faire passer un échantillon de gaz cible dans un canal primaire, dans lequel un convertisseur primaire convertit des composés d'azote gazeux contenus dans l'échantillon de gaz cible dans un gaz indicateur, et un détecteur primaire génère une indication du gaz indicateur;
faire passer un échantillon de gaz de référence dans un canal d'épuration, dans lequel l'épurateur retire des composés d'azote basiques, et un convertisseur secondaire convertit des composés d'azote gazeux dans l'échantillon de gaz de référence dans un gaz indicateur, et un détecteur secondaire génère une indication du gaz indicateur; et
comparer les indications fournies par le détecteur primaire et par le détecteur secondaire pour fournir une indication concernant la concentration des composés d'azote de base sur la base de la différence d'indications des détecteurs.

8. Procédé selon la revendication 7, selon lequel des indications enregistrées simultanément et fournies par le détecteur primaire et le détecteur secondaire sont comparées.

9. Système de détection pour détecter des composés d'azote basiques comprenant :

un dispositif à convertisseurs qui convertit un composé d'azote contenu dans un échantillon de gaz en un gaz indicateur;
un capteur qui détecte le gaz indicateur, le capteur étant couplé au dispositif à convertisseurs pour recevoir un échantillon de gaz de la part du dispositif à convertisseurs;
un canal primaire couplé au dispositif à convertisseurs pour délivrer un échantillon de gaz ciblé au dispositif à convertisseurs;
au moins un canal d'épuration incluant au moins un épurateur, le canal d'épuration étant couplé au dispositif à convertisseurs pour délivrer un échantillon de gaz de référence au dispositif à convertisseurs; et
un troisième canal couplé au capteur pour délivrer un échantillon de gaz non épuré non converti au capteur.

10. Système de détection selon la revendication 9,

a) dans lequel le capteur inclut une pluralité de détecteurs de telle sorte que l'ensemble des trois canaux n'utilisent pas un détecteur commun; ou
b) dans lequel le dispositif à convertisseurs inclut au moins deux convertisseurs, des convertisseurs distincts étant positionnés dans le canal primaire et dans le canal d'épuration; ou

c) dans lequel le canal d'épuration inclut une paire de dispositifs d'épuration réuni en parallèle.

11. Système de détection pour détecter un gaz contaminant, comprenant :

un capteur qui détecte un gaz indicateur dans un échantillon de gaz;
un canal primaire qui délivre un échantillon de gaz au capteur;
une pluralité de canaux d'épuration qui délivrent un échantillon de gaz de référence au détecteur de telle sorte que chacun des canaux d'épuration comprend un épurateur qui retire un composé d'azote basique de l'échantillon de gaz de référence; et
un convertisseur couplé au canal primaire et aux canaux d'épuration et qui convertit un composé d'azote gazeux contenu dans un échantillon de gaz dans le gaz indicateur.

12. Système de détection selon la revendication 11, et

a) comprenant en outre un système de purge couplé au moins à l'un des épurateurs pour purger des composants d'azote basiques liés de façon réversible, hors de l'épurateur, ou
b) dans lequel les épurateurs incluent un milieu d'échange de cations; ou
c) dans lequel les canaux d'épuration sont connectés en parallèle; et facultativement,

comprenant en outre un dispositif de commande d'écoulement positionné de manière à commander celui des canaux d'épuration dans lequel le gaz échantillonné peut circuler en direction du convertisseur, auquel cas, facultativement,

le dispositif de commande d'écoulement est commandé par un système de commande qui est programmé pour transférer l'écoulement de l'échantillon de gaz de référence, qui atteint le capteur, depuis un canal d'épuration comportant un épurateur pollué à un canal d'épuration comportant un épurateur purgé, puis diriger un gaz de purge à travers l'épurateur pollué, auquel cas, facultativement,

le système de commande est programmé pour transférer l'écoulement de l'échantillon de gaz de référence à partir de l'un des canaux d'épuration et de purger l'épurateur de ce canal d'épuration avant qu'un composé d'azote faiblement basique puisse pénétrer dans l'épurateur; ou

le système de commande est programmé pour transférer alternativement l'écoulement de l'échantillon de gaz entre le canal primaire, dans lequel l'échantillon de gaz devient un échantillon de gaz cible, et l'un des canaux d'épuration, dans lequel l'échantillon de gaz devient un échantillon de gaz de référence; ou
d) dans lequel les épurateurs incluent des billes recouvertes d'un photoresistant; ou
e) le système de détection comprend en outre un réducteur de pression situé entre le capteur et les épurateurs; ou
f) les épurateurs sont exclus du canal primaire.

13. Procédé de contrôle d'une contamination par des composés d'azote basiques dans un gaz échantillonné, comprenant les étapes consistant à :

faire passer un premier échantillon de gaz de référence dans un premier épurateur pour éliminer un composé d'azote basique du premier échantillon de gaz de référence;
faire passer un second échantillon de gaz de référence dans un second épurateur pour éliminer un composé d'azote basique du second échantillon de gaz de référence;
purger le premier épurateur pour retirer des composés d'azote basiques liés, alors que le second échantillon de gaz de référence traverse le second épurateur;
faire passer les premier et second échantillons de gaz de référence dans un convertisseur qui convertit le composé d'azote gazeux contenus dans les échantillons de gaz de référence dans un gaz indicateur après que les échantillons de gaz de référence aient traversé leurs épurateurs respectifs;
faire passer les premier et second échantillons de gaz de référence dans un capteur qui détecte une concentration du gaz indicateur dans les échantillons de gaz de référence une fois que les échantillons de gaz de référence aient traversé le convertisseur;
faire passer un échantillon de gaz cible dans le convertisseur, qui convertit des composés d'azote gazeux contenus dans l'échantillon de gaz cible dans le gaz indicateur;
faire passer l'échantillon de gaz cible dans le capteur qui détecte la concentration du gaz indicateur en l'échantillon de gaz cible après que l'échantillon de gaz cible ait traversé le convertisseur;
déterminer une concentration de pollution par des composés d'azote basiques par comparaison de la concentration détectée du gaz indicateur dans l'échantillon de gaz cible à la concentration détectée du gaz indicateur dans au moins l'un des échantillons de gaz de référence.

14. Procédé selon la revendication 13 et

a) dans lequel un dispositif de détection commun détecte alternativement la concentration des gaz indicateurs dans l'échantillon de gaz de référence circulant dans le premier épurateur et la concentration du gaz indicateur dans l'échantillon de gaz de référence circulant dans le second épurateur; ou

b) comprenant en outre l'étape de purge alternée des premier et second épurateurs, tout en maintenant un écoulement essentiellement ininterrompu des échantillons de gaz envoyés au convertisseur et au capteur; ou

c) dans lequel les échantillons de gaz sont tirés d'un groupe d'outils de photolithographie.

**15.** Système de photolithographie comprenant :

un outil de photolithographie;
un capteur qui détecte une concentration d'un gaz indicateur dans un échantillon de gaz provenant de l'outil de photolithographie;
un canal primaire qui délivre un échantillon de gaz cible au détecteur;
une pluralité de canaux d'épuration servant à délivrer un échantillon de gaz de référence au détecteur de telle sorte que chacun des canaux d'épuration inclut un épurateur qui retire un composé d'azote de base de l'échantillon de gaz de référence;
au moins un convertisseur couplé au canal primaire et aux canaux d'épuration pour convertir des composés d'azote gazeux contenus dans les échantillons de gaz dans le gaz indicateur; et
un système de commande qui commande l'écoulement d'un échantillon de gaz entre le canal primaire et les canaux d'épuration.

**16.** Système selon la revendication 14, comprenant en outre un système de purge couplé à chacun des épurateurs qui purgent des composés d'azote basiques retirés, à partir de chacun des épurateurs, les épurateurs comprenant des milieux d'échange d'ions.

FIG. _1

FIG._2

FIG._3

FIG._4

FIG._5

6

CONVERSION
MODULE

7

NO
DETECTOR

SAMPLE
IN →

22

VACUUM
PUMP

FIG. 6

NO

124

158

POWER
RELAY
CONTROLLER

152   154

150

156

FIG._8D

$O_2, N \cdot X$

FIG. _7

EP 1 144 995 B1

FIG. _8A

EP 1 144 995 B1

FIG._8B

EP 1 144 995 B1

FIG._8C

FIG._8E

EP 1 144 995 B1

6

118c                116c

118b     116b    A          120
                          SCRUBBER
                          SYSTEM
                    140
                 B              116a

116a

PRESSURE          THERMAL
REDUCER           CATALYTIC
                  CONVERTER
   118    122              124    116

130
CONTROL
SYSTEM                    → TO DETECTOR

FIG._9

EP 1 144 995 B1

CHANNEL 1

120
SCRUBBER
SYSTEM

PRESSURE
REDUCER

124a
THERMAL
CATALYTIC
CONVERTER

126a
CHEMI-
LUMINESCENT
NO DETECTOR

114a

CHANNEL 2

PRESSURE
REDUCER

124b
THERMAL
CATALYTIC
CONVERTER

126b
CHEMI-
LUMINESCENT
NO DETECTOR

114b

130'
CONTROL
SYSTEM

FIG._10

FIG. 11

EP 1 144 995 B1

ZERO/KNOWN-
CONCENTRATION
SAMPLES

CONVERTER —124

DETECTOR —126

KNOWN ZERO
CONCENTRATION
READING

CALIBRATED
SPAN CONCEN-
TRATION READING

CALCULATE
DIFFERENCE($\Delta$)
IN DETECTOR
READINGS

CORRELATE $\Delta$
IN DETECTOR
READINGS WITH
$\Delta$ IN SPAN

S

FIG. 12

44

PROCESS GAS SAMPLE → SCRUBBER → CONVERTER → DETECTOR —126

120    124

APPLY ZERO CALIBRATION

APPLY INTERFER-ENCE CALIBRATION

(S) → APPLY SPAN CALIBRATION

GENERATE SCALED VALUE FOR CON-TAMINATION LEVEL

IS CONTAMIN-ATION LEVEL >0?

CONTINUE OPERATION ← NO

YES

IS CONTAMIN-ATION LEVEL >THRESH-OLD?

SCHEDULE FILTER CHANGE ← NO

YES

TERMINATE PROCESS

FIG. 13

FIG. 14

| View | Options | Help |
|------|---------|------|
| ✓Toolbar | | |
| ✓Status Bar | | |

Diagnostic Display

Stepper
Interface
PEB Area
Wafer Loader
Stepper Filter
Track Filter
Filter Interstack
Bay 8 Ambient
Service Chase
Coat Bowl2

## FIG. 15

| Print | Diagnostic Display | Jump to Location |
|-------|--------------------|------------------|

Open File

Help

Save Settings | Journal Entry | Hold/Sequence Mode

## FIG. 16

**System Definition** ⊠

| Data Acquisition Scheduling | Sample Locations | Local Alarms | Calibration | Security |

| Group Label | Status | Execution Mode | |
|---|---|---|---|
| Critical | Disabled | Repeatedly (priority 2) | Add Group... |
| Occasional | | Scheduled (6.67 hours) | Change Group... |
| calibration | | Repeatedly (priority 5) | Remove Group... |

| Step # Status | Location ID | Purge Time | Sample Time |
|---|---|---|---|
| Step 1 | Location 9 | 10.0 min (600 s) | 10.0 min (600 s) |
| Step 2 | Location 10 | 10.0 min (600 s) | 10.0 min (600 s) |

| Add Step... | Number of Steps in Group | 2 |
|---|---|---|
| Change Step... | Active Steps | 2 |
| Remove Step... | Group Duration* | 2400 | secs ▽ |

*Actual timing may vary with operating conditions.

| OK | Cancel |

# FIG. 17

FIG. 18

System Definition ☒

| Data Acquisition Scheduling | Sample Locations | Local Alarms | Calibration | Security |

┌ Local Alarms ─────────────────────────────

| Location ID | Hi Warning | Hi Alarm | Fault |
|---|---|---|---|
| Location 1 | 30.0 ppb | 40.0 ppb | Enabled |
| Location 2 | 30.0 ppb | 40.0 ppb | Enabled |
| Location 3 | 30.0 ppb | 40.0 ppb | Enabled |
| Location 4 | 30.0 ppb | 40.0 ppb | Enabled |
| Location 5 | 30.0 ppb | 40.0 ppb | Enabled |
| Location 6 | 30.0 ppb | 40.0 ppb | Enabled |

Edit Alarms...

Recent event indicator time interval (for system (if 0, can only be cleared manually)    6.00  hours ▽

┌ Digital Outputs ───────────────────────────

| Output | Active | Override | Label |
|---|---|---|---|
| A | High | No | Digital Output A Control |
| B | High | No | Digital Output B Control |
| C | High | No | Digital Output C Control |
| D | High | No | Digital Output D Control |

Edit Outputs...

OK    Cancel

FIG. 19

SAMPLE ─── SCRUBBER SYSTEM /120 ─── IMS /170

FIG. 21

**Location 10 - Location 10** [_][□][×]

Sampling location: Location 10   ——— alarm threshold    ----- warming threshold

Real Time View

ppb Total Molecular Base

50
40
30
20
10
0

1/03 10:59   1/03 22:59   1/04 10:59   1/04 22:59   1/05 10:59   1/05 22:59   1/06 10:59

RT

Last Reading [    ] ppb

Alarm Threshold [40.0] ppb

Warning Threshold [30.0] ppb

— Viewing Area —
Y-Axis Scale (ppb)

[50] [▲▼]

Y-Axis Interval

[72.0] [hours] [▼]

☑ Show Grid
☐ Line Plot

[ Apply ]

— Journal —

[▲]
[▼]

[ New ]

[ Print Graph ]

[ Save Setting ]

[ Close ]

FIG. 20